# EUROPEAN PATENT SPECIFICATION

(11) **EP 3 863 682 B1**
(45) Date of publication and mention of the grant of the patent: **17.05.2023**
(21) Application number: 19783071.4
(22) Date of filing: 11.10.2019
(51) Int. Cl.: A61K 48/00, C12N 15/09, C12N 15/86

(54) **CODON-OPTIMIZED TRANSGENE FOR THE TREATMENT OF PROGRESSIVE FAMILIAR INTRAHEPATIC CHOLESTASIS TYPE 3 (PFIC3)**
CODON-OPTIMIERTES TRANSGEN FÜR DIE BEHANDLUNG VON PROGRESSIVER FAMILIEN INTRAHEPATISCHER CHOLESTASE TYP 3 (PFIC3)
TRANSGENE OPTIMISÉ PAR CODON POUR LE TRAITEMENT DE LA CHOLESTASE INTRAHÉPATIQUE FAMILIALE PROGRESSIVE DE TYPE 3 (PFIC3)

(30) Priority: 12.10.2018 EP 18306349
(43) Date of publication of application: 18.08.2021
(60) Divisional application: 23166123.2
(73) Proprietor: Vivet Therapeutics, 75008 Paris (FR)
(72) Inventor: WEBER, Nicholas, 31012 PAMPLONA (ES); GONZALEZ ASEGUINOLAZA, Gloria, 31010 Barañain (ES); SMERDOU, Cristian, 31009 PAMPLONA (ES)
(74) Representative: Plasseraud IP
(86) International application number: PCT/EP2019/077568
(87) International publication number: WO 2020/074690

(56) References cited:
- US-A1- 2017 216 456
- DE VREE J MARLEEN L ET AL: "Correction of liver disease by hepatocyte transplantation in a mouse model of progressive familial intrahepatic cholestasis", GASTROENTEROLOGY, vol. 119, no. 6, December 2000 (2000-12), pages 1720-1730, XP029482084, ISSN: 0016-5085, DOI: 10.1053/GAST.2000.20222
- JACQUEMIN EMMANUEL: "Progressive familial intrahepatic cholestasis", CLINICS AND RESEARCH IN HEPATOLOGY AND GASTROENTEROLOGY, ELSEVIER MASSON, FR, vol. 36, no. Suppl. 1, 31 August 2012 (2012-08-31), pages S26-S35, XP009509855, ISSN: 2210-7401, DOI: 10.1016/S2210-7401(12)70018-9
- THOMAS FALGUIÈRES ET AL: "ABCB4: Insights from pathobiology into therapy", CLINICS AND RESEARCH IN HEPATOLOGY AND GASTROENTEROLOGY, vol. 38, no. 5, 1 October 2014 (2014-10-01), pages 557-563, XP055530418, FR ISSN: 2210-7401, DOI: 10.1016/j.clinre.2014.03.001
- SIEW S M SIEW S M ET AL: "Long-term disease correction and reduced liver tumorigenesis using hybrid recombinant AAV-PIGGYBAC transposon mediated gene therapy in the progressive familialintrahepaticcholestasis type 3 (PFIC3) mouse model in vivo", HEPATOLOGY, WILEY INTERSCIENCE, US, vol. 66, no. Supplement 1, 1 October 2017 (2017-10-01), pages 365A-366A, XP009509853, ISSN: 1527-3350

## Description

### FIELD OF THE INVENTION

The present disclosure relates to gene therapy vector for use in the treatment of progressive familiar intrahepatic cholestasis type 3. More specifically, the present invention relates to an adeno-associated virus vector comprising codon-optimized sequence encoding for the MDR3 isoform A for the treatment of PFIC3.

### BACKGROUND ART

Progressive familial intrahepatic cholestasis type 3 (PFIC3) is a genetic disease associated with mutations in adenosine triphosphate-binding cassette, subfamily B, 4 (ABCB4) gene, coding for multidrug resistance protein 3 (MDR3) (Jacquemin E. Clin Res Hepatol Gastroenterol. 2012; 36 Suppl 1:526-35). This protein, which is expressed predominantly in the canalicular membrane of hepatocytes, is a floppase involved in the translocation of phosphatidylcholine from the hepatocyte membrane to the bile. Phosphatidylcholine is necessary to neutralize the toxicity of bile salts through the generation of mixed micelles. Mutations in ABCB4 gene prevent proper micelle formation, resulting in bile canaliculi and biliary epithelium injury, leading to cholestasis (Jacquemin E. et al. Gastroenterology. 2001; 120:1448-1458).

There is currently no cure for PFIC3, and therefore the unmet medical need is very high. Although ursodeoxycholic acid (UDCA) therapy may ameliorate symptoms in some patients, outside of liver transplant there is currently no curing treatment for PFIC3. Surgical intervention in the form of biliary diversion improves patient outcomes. However, post-surgical complications such as infections and issues with stoma bags impact patients' quality of life, while the risk of cirrhosis and liver cancer still remains. Liver transplants are an effective treatment, but carry with them the risks involved with such a complicated procedure as well as a chance of re-emergence of the condition (van der Woerd WL et al. World J gastroenterol. 2017; 23(5):763-775).

Gene therapy correcting the defective gene responsible for disease development is a promising treatment for a number of diseases. However, the technique remains still under study. Stable integration and expression of ABCB4 in liver cells to treat or prevent PFIC3 is disclosed in WO2015/139093. However, these integrating vector systems present a main disadvantage that is their potential risk of causing insertional mutagenesis. RNA therapy to treat a liver condition such as progressive familial intrahepatic cholestatsis 3 (PFIC3) using various potential therapeutic genes including ABCB4 was only suggested in WO2017/100551. Thus, there is still a need to develop gene therapy methods which avoid insertional mutagenesis and meanwhile allow stable and long term transgene expression. It is described herein a PFIC3 gene therapy with codon-optimized sequences encoding MDR3 isoform A that allows successful high expression in cholestatic liver so as to revert the cause of the toxicity in the bile of affected patients.

### SUMMARY OF THE INVENTION

Surprisingly, the inventors found that contrary to the wild type MDR3 isoform A, the codon optimized sequence of MDR3 isoform A when administered *in vivo* showed an efficient expression specifically in the canalicular membranes of hepatocytes. In Abcb4^{-/-} knock-out mice which reproduce most of PFIC3 symptoms, administration of AAV encoding codon optimized versions of MDR3 isoform A achieve a long-term therapeutic effect such as significant restoration of PFIC3 serum biomarker levels, decrease of liver and spleen size, increase of bile phosphatidylcholine and correction of the liver morphology abnormalities.

A first aspect of the present disclosure thus relates to a nucleic acid construct comprising a transgene encoding MDR3 isoform A, said transgene being represented by SEQ ID NO: 1 or a sequence having at least 90 % of identity with SEQ ID NO: 1.

In specific embodiments, said nucleic acid construct further comprises a promoter which initiates transgene expression upon introduction into a host cell, preferably a liver specific promoter, more preferably an alpha-1-antitrypsin promoter or a bile salt-inducible promoter. In specific embodiments, said vector further comprises a polyadenylation signal sequence, for example a synthetic polyadenylation signal sequence having sequence SEQ ID NO: 3.

In specific embodiments, said nucleic acid construct further comprises a 5'ITR and a 3'ITR sequences, preferably a 5'ITR and a 3'ITR sequences of adeno-associated virus (AAV), notably a 5'ITR and a 3'ITR sequences from the AAV2 serotype.

In more specific embodiments, said nucleic acid construct comprises or consists of a nucleic acid sequence SEQ ID NO: 4 or a nucleic acid sequence having at least 90% of identity with SEQ ID NO: 4.

In another aspect, said nucleic acid construct is comprised in an expression vector, preferably a viral vector, more preferably an AAV vector.

Another aspect of the present disclosure relates to a viral particle comprising a nucleic acid construct or an expression vector of the invention, and preferably comprising capsid proteins of adeno-associated virus such as capsid proteins selected from the group consisting of AAV3 type 3A, AAV3 type 3B, NP40, NP59, NP84, LK03, AAV3-ST, Anc80 and AAV8 serotype.

Another aspect of the present disclosure relates to a host cell comprising the nucleic acid construct or the expression vector of the invention, or a host cell transduced with a viral particle of the invention.

Another aspect of the present disclosure relates to a pharmaceutical composition comprising the nucleic acid construct, expression vector, host cell, or viral particle of the invention, in combination with one or more pharmaceutical acceptable excipient, diluent or carrier, optionally comprising other active ingredients.

The invention also relates to a product of the invention for use as a medicament, such as the prevention and/or the treatment of progressive familial intrahepatic cholestasis type 3 in a subject in need thereof. In a specific embodiment, the subject is a neonate, an infant, a child or an adult, preferably a neonate, an infant or a child, more preferably a neonate or an infant.

Also disclosed herein is a process for producing viral particles as described above, comprising the steps of: a) culturing a host cell as described above in a culture medium, and b) harvesting the viral particles from the cell culture supernatant and/or inside the cells.

The present disclosure also relates to a kit comprising the nucleic acid construct, expression vector, host cell, viral particle, or pharmaceutical composition as described above, in one or more containers, optionally further comprising instructions or packaging materials.

### BRIEF DESCRIPTION OF THE DRAWINGS

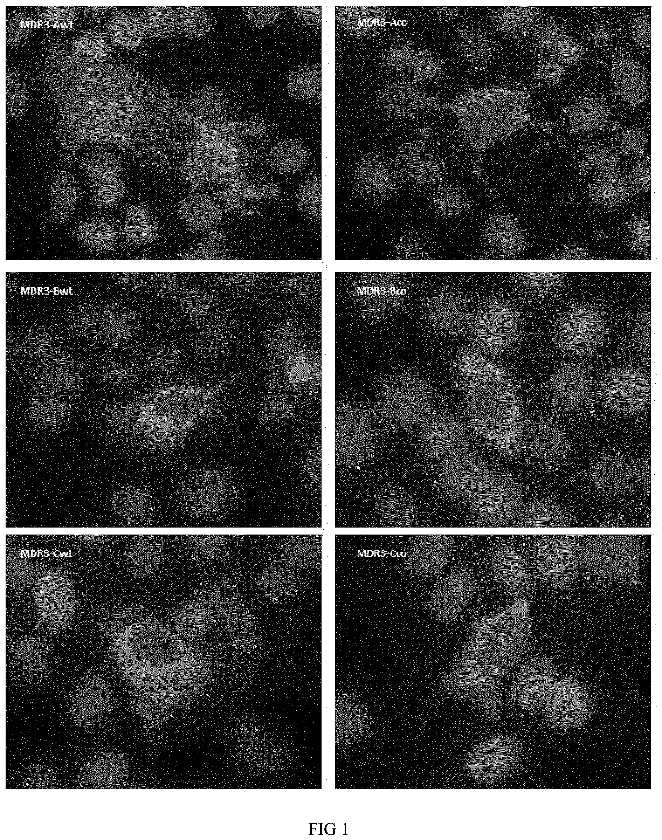

**Figure 1****:** Immunofluorescence microscopy images of Huh7 cells transfected with plasmids expressing the indicated human MDR3 isoforms. Wt: wild type sequence, co: codon-optimized sequence. Nuclei were stained with DAPI.

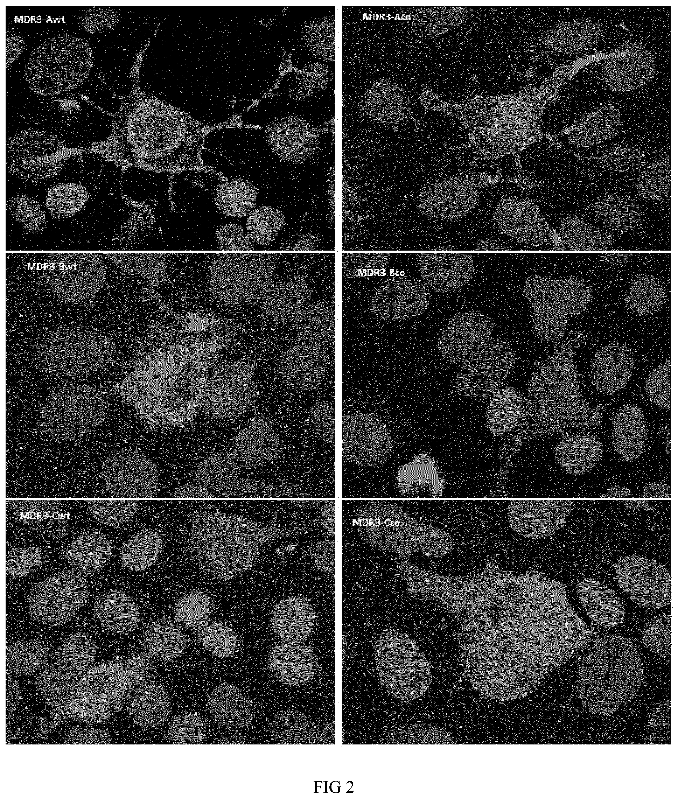

**Figure 2****:** Confocal microscopy images of Huh7 cells transfected with plasmids expressing the indicated human MDR3 isoforms. For isoforms Bwt, Bco, Cwt, and Cco orthogonal projections are shown to demonstrate cytoplasmic localization of MDR3. wt: wild type sequence, co: codon-optimized sequence. Nuclei were stained with DAPI.

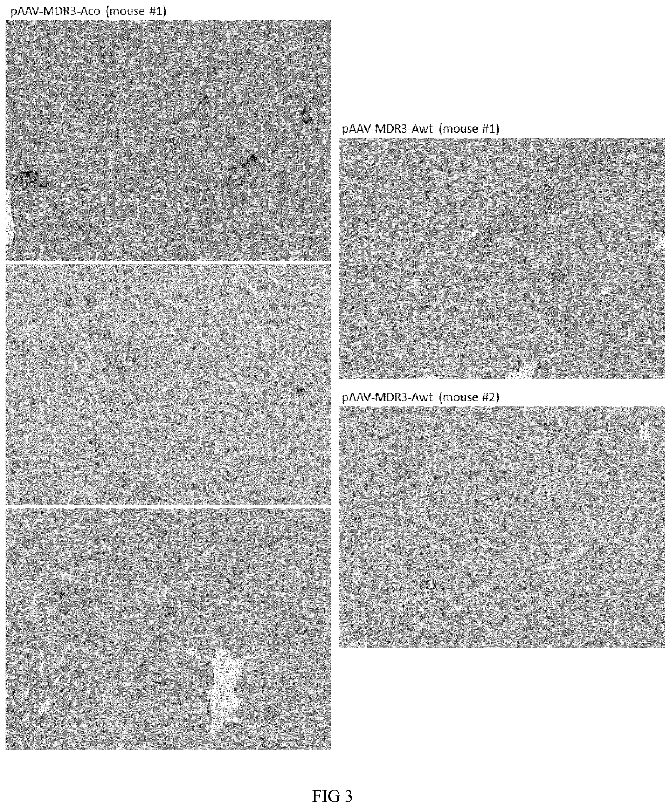

**Figure 3****:** MDR3 expression in liver sections from mice injected with pAAV-MDR3 plasmids via HDI (naked DNA transfer). Three images from one mouse injected with MDR3 isoform Aco are shown (left panels), and both mice injected with MDR3 isoform Awt are shown (right panels).

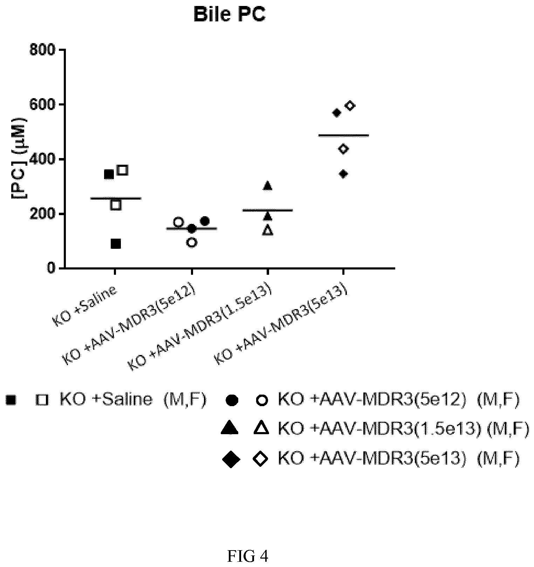

**Figure 4****:** Bile PC concentration in 3 week-old Abcb4^{-/-} mice treated with AAVAnc80-MDR3-Aco vectors. AAV doses are indicated below the x axis. Males (M) are filled symbols and females (F) are open symbols.

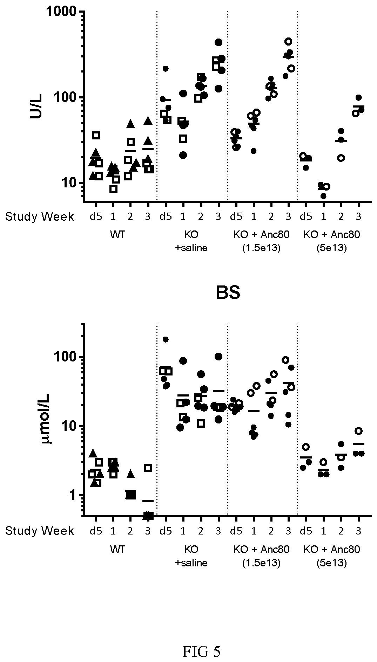

**Figure 5****:** Serum alanine transaminase (ALT) and bile salt (BS) levels in AAVAnc80-MDR3-Aco-treated Abcb4^{-/-} mice. Males are indicated in filled symbols, females in open symbols. Samples were collected at 5 days, 1, 2 and 3 weeks post-treatment, d, days; WT, Abcb4^{+/+} mice; KO, Abcb4^{-/-} mice.

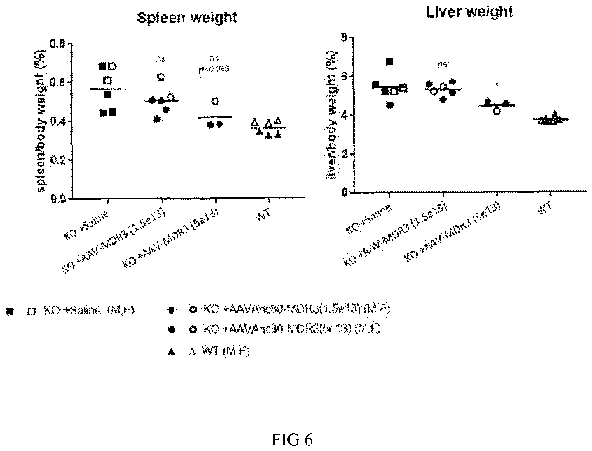

**Figure 6****:** Spleen and liver weights (as a percentage of body weight) of Abcb4^{-/-} mice (KO) treated with AAVAnc80-MDR3-Aco at 2 weeks of age and sacrificed 3 weeks later compared to untreated ABCB4^{-/-} (squares) or wild-type (wt) mice (triangles). Males (M) are indicated in filled symbols, females (F) in open symbols. *, p<0.05; ns, not significant.

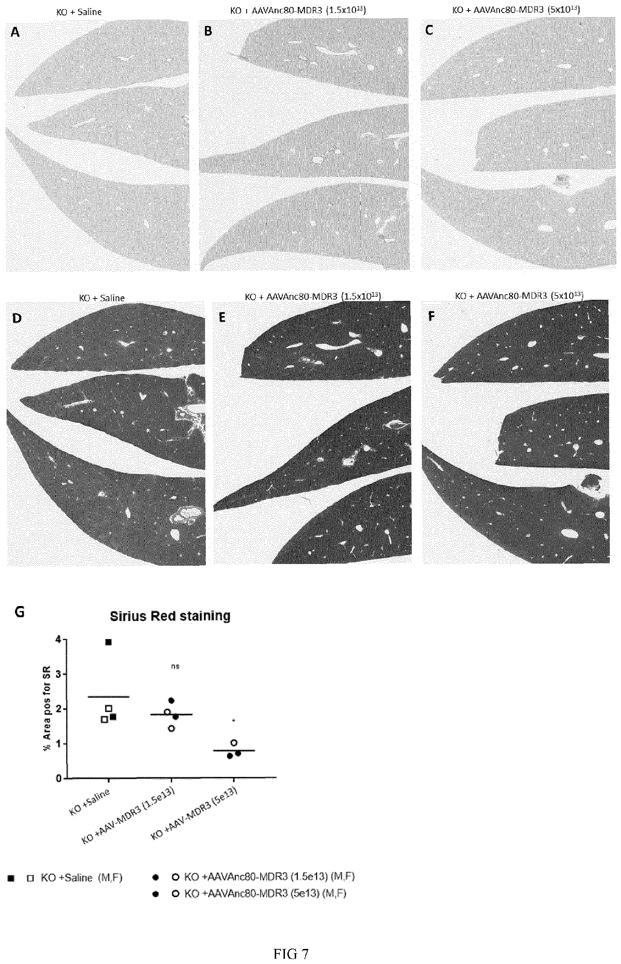

**Figure 7****:** Sirius Red (A-C) and Masson's Trichrome (D-F) staining of liver in male Abcb4^{-/-} mice (KO) treated at 2 weeks of age with saline (A & D) or AAVAnc80-MDR3-Aco at 1.5×10¹³ (B & E) or 5×10¹³ (C & F) and sacrificed 3 weeks later. (G) Quantification of percent area positive for Sirius Red staining was performed via Imaged software. *: p<0.05; ns: not significant.

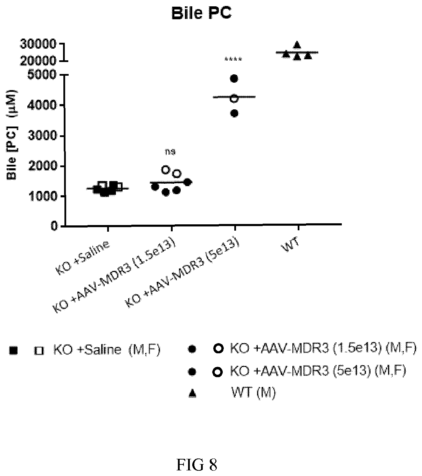

**Figure 8****:** Bile PC concentration of Abcb4^{-/-} mice (KO) treated with AAVAnc80-MDR3-Aco at 2 weeks of age and sacrificed 3 weeks later compared to untreated Abcb4^{-/-} (squares) or wt mice (triangles). Males (M) are indicated in filled symbols, females (F) in open symbols. ^{∗∗∗∗}, p<0.0001; ns, not significant.

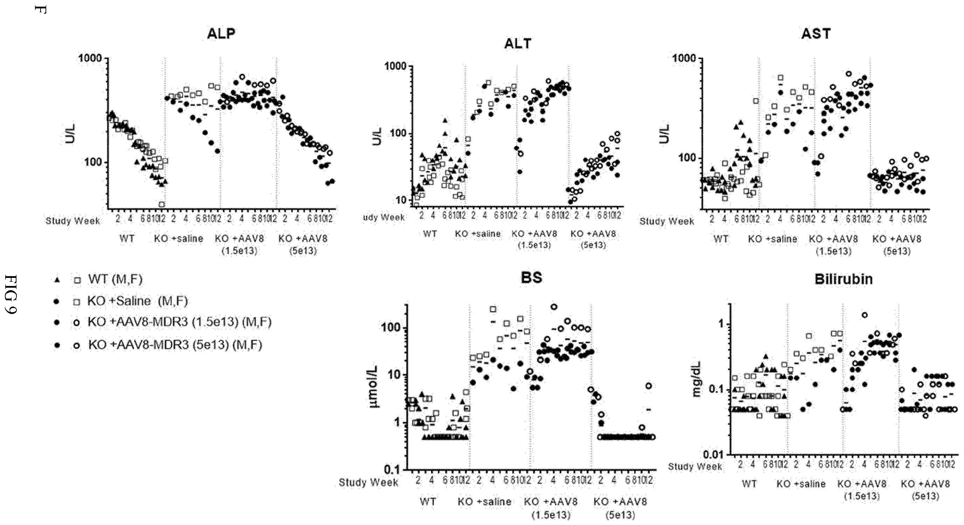

**Figure 9****:** Serum biomarkers in Abcb4^{-/-} mice treated with AAV8 -MDR3-Aco. Males (M) are indicated in filled symbols, females (F) in open symbols. Weeks since treatment are indicated immediately below the x axis, and AAV doses for each group are indicated below.

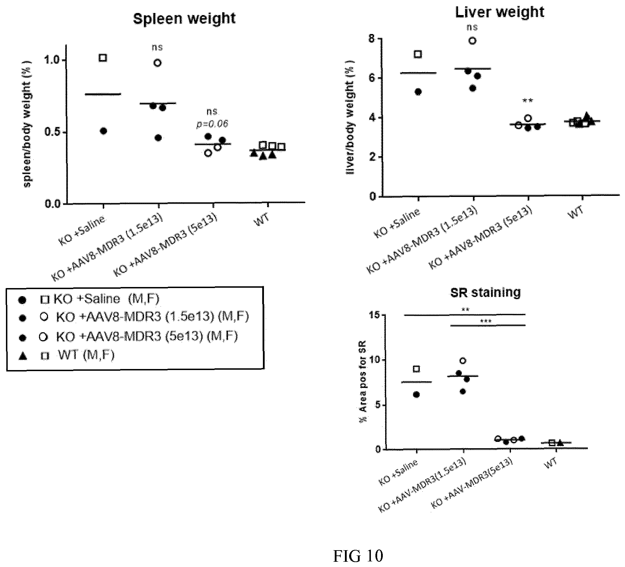

**Figure 10****:** Spleen and liver weights (as a percentage of body weight) of Abcb4^{-/-} mice (KO) treated with AAV8-MDR3-Aco and sacrificed 12 weeks later compared to untreated ABCB4^{-/-} (KO) or wild-type (wt) mice. Males (M) are indicated in filled symbols, females (F) in open symbols. ***, p<0.001 **, p<0.01; ns, not significant.

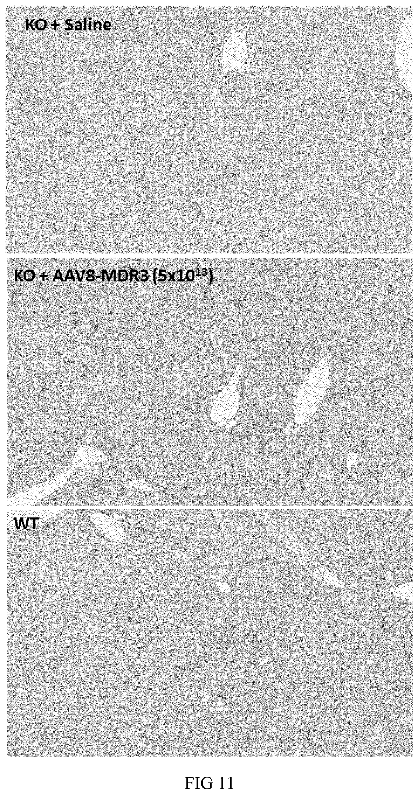

**Figure 11****:** IHC staining with anti-MDR3 antibody of liver sections from Abcb4^{-/-} mice treated with saline (top) or AAV8-MDR3-Aco at 5×10¹³ VG/kg (middle) and harvested one week later. Staining of a wild type mouse (WT) liver section is included as a comparator and positive control (bottom).

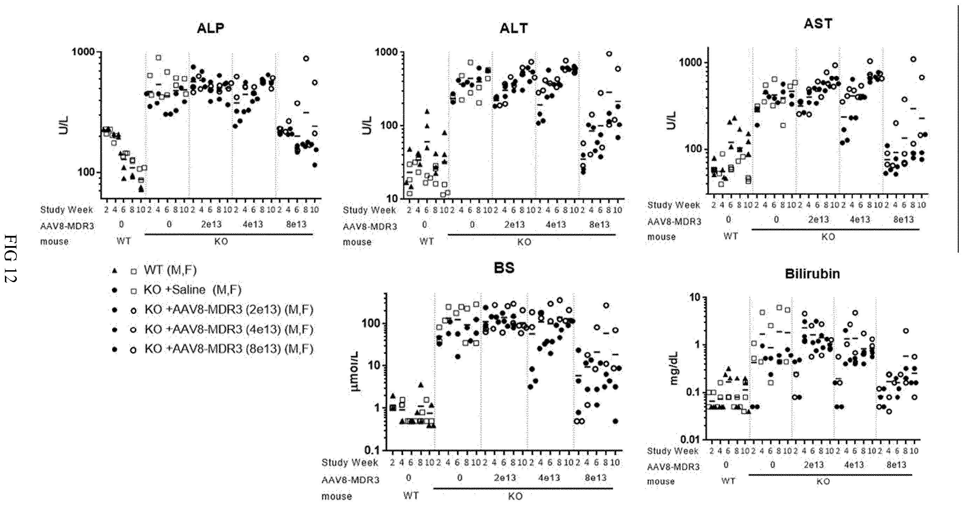

**Figure 12****:** Serum biomarker levels for a dose range finding study of AAV8-MDR3-Aco in Abcb4^{-/-} mice. The indicated serum markers were analyzed 1 to 10 weeks after vector administration. Males (M) are indicated in filled symbols, females (F) in open symbols. AAV doses are indicated below the x axis.

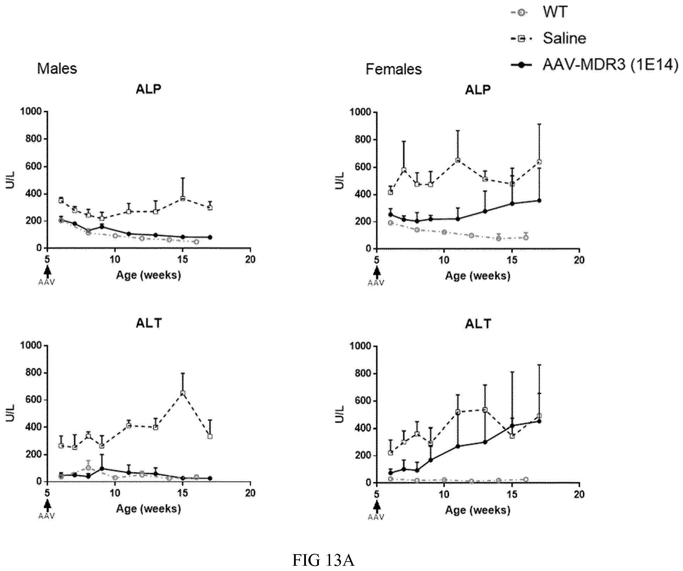

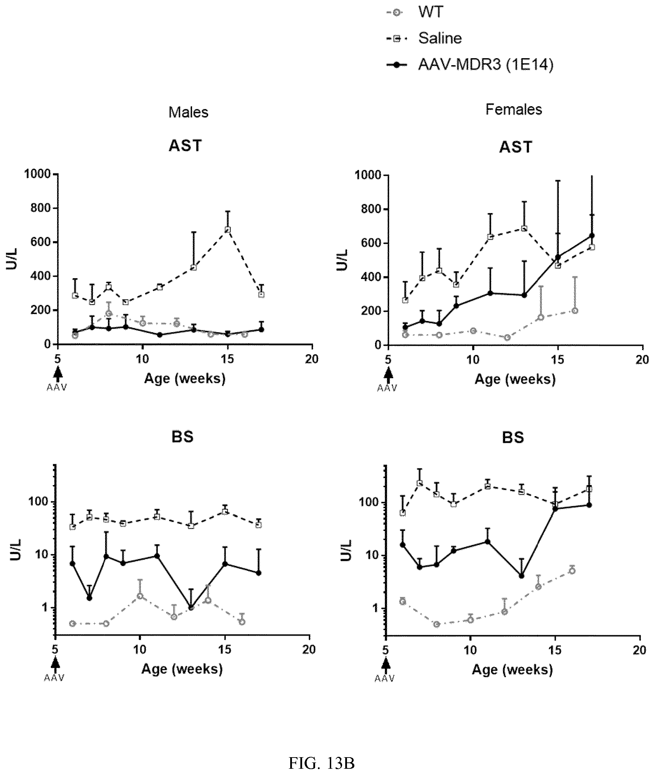

**Figure 13****:** A) Serum alkaline phosphatase (ALP), alanine transaminase (ALT), B) aspartate transaminase (AST), and bile salt (BS) levels from Abcb4^{-/-} mice treated at 5 weeks of age. Wild-type (WT) animals are indicated with grey open circles, saline-treated Abcb4^{-/-} mice are indicated with black squares, and AAV-MDR3-Aco-treated Abcb4^{-/-} mice are indicated with black filled circles. Males are shown in graphs on the left and females on the right.

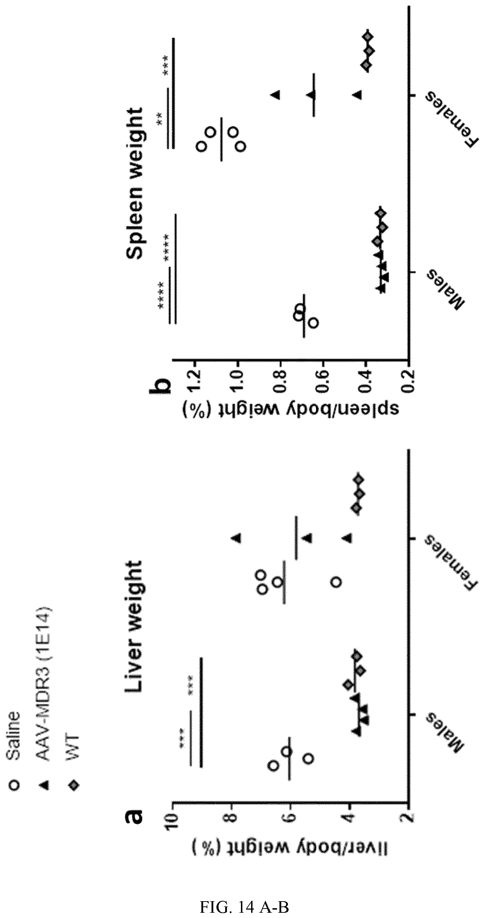

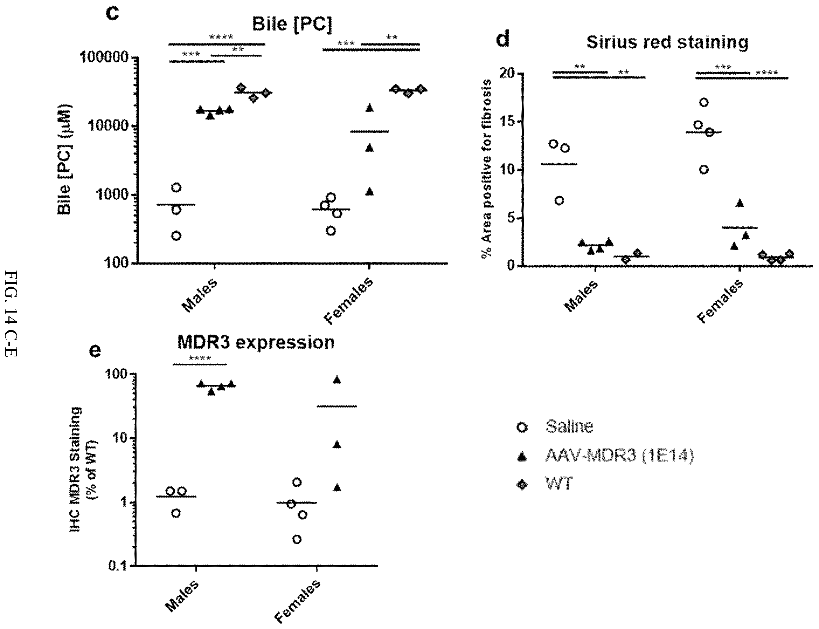

**Figure 14****:** Liver (a) and spleen (b) weights (as a percentage of body weight), bile PC (c), fibrosis (d), and MDR3 protein expression (e) in Abcb4^{-/-} mice treated at 5 weeks of age. Wild-type (WT) animals are indicated with grey diamonds, saline-treated Abcb4^{-/-} mice are indicated with open circles, and AAV-MDR3-Aco-treated Abcb4^{-/-} mice are indicated with black triangles.

### DETAILED DESCRIPTION

The invention relates to a transgene comprising a codon-optimized sequence encoding MDR3 isoform A (NCBI reference sequence: NP_000434.1). The membrane-associated protein encoded by ABCB4 gene, also named MDR3 gene is a member of the superfamily of ATP-binding cassette (ABC) transporters. This gene encodes a full transporter and member of the p-glycoprotein family of membrane proteins with phosphatidylcholine as its substrate which may be involved in transport of phospholipids from liver hepatocytes into bile. Alternative splicing of this gene results in three potential isoforms, designated A, B and C.

As used herein, the term "transgene" refers to exogenous DNA or cDNA encoding a gene product. The gene product may be an RNA, peptide or protein. In addition to the coding region for the gene product, the transgene may include or be associated with one or more elements to facilitate or enhance expression, such as a promoter, enhancer(s), response element(s), reporter element(s), insulator element(s), polyadenylation signal(s) and/or other functional elements. Embodiments of the invention may utilize any known suitable promoter, enhancer(s), response element(s), reporter element(s), insulator element(s), polyadenylation signal(s) and/or other functional elements. Suitable elements and sequences will be well known to those skilled in the art.

### Nucleic acid construct

More particularly, the invention relates to a nucleic acid construct comprising a transgene encoding MDR3 isoform A, said transgene being represented by SEQ ID NO: 1 or 2 or having at least 90% identity with SEQ ID NO: 1 or 2.

The terms "nucleic acid sequence" and "nucleotide sequence" may be used interchangeably to refer to any molecule composed of or comprising monomeric nucleotides. A nucleic acid may be an oligonucleotide or a polynucleotide. A nucleotide sequence may be a DNA or RNA. A nucleotide sequence may be chemically modified or artificial. Nucleotide sequences include peptide nucleic acids (PNA), morpholinos and locked nucleic acids (LNA), as well as glycol nucleic acids (GNA) and threose nucleic acid (TNA). Each of these sequences is distinguished from naturally-occurring DNA or RNA by changes to the backbone of the molecule. Also, phosphorothioate nucleotides may be used. Other deoxynucleotide analogs include methylphosphonates, phosphoramidates, phosphorodithioates, N3'P5'-phosphoramidates and oligoribonucleotide phosphorothioates and their 2'-0-allyl analogs and 2'-0-methylribonucleotide methylphosphonates which may be used in a nucleotide of the invention.

The term "nucleic acid construct" as used herein refers to a man-made nucleic acid molecule resulting from the use of recombinant DNA technology. A nucleic acid construct is a nucleic acid molecule, either single- or double-stranded, which has been modified to contain segments of nucleic acids sequences, which are combined and juxtaposed in a manner, which would not otherwise exist in nature. A nucleic acid construct usually is a "vector", i.e. a nucleic acid molecule which is used to deliver exogenously created DNA into a host cell.

As used herein, the term "sequence identity" or "identity" refers to the number of matches (identical nucleic acid residues) in positions from an alignment of two polynucleotide sequences. The sequence identity is determined by comparing the sequences when aligned so as to maximize overlap and identity while minimizing sequence gaps. In particular, sequence identity may be determined using any of a number of mathematical global or local alignment algorithms, depending on the length of the two sequences. Sequences of similar lengths are preferably aligned using a global alignment algorithms (e.g. Needleman and Wunsch algorithm; Needleman and Wunsch, 1970, J Mol Biol.;48(3):443-53) which aligns the sequences optimally over the entire length, while sequences of substantially different lengths are preferably aligned using a local alignment algorithm (e.g. Smith and Waterman algorithm (Smith and Waterman, 1981, J Theor Biol. ;91(2):379-80) or Altschul algorithm (Altschul SF et al., 1997, Nucleic Acids Res.;25(17):3389-402.; Altschul SF et al., 2005, Bioinformatics. ;21(8):1451-6)). Alignment for purposes of determining percent nucleic acid sequence identity can be achieved in various ways that are within the skill in the art, for instance, using publicly available computer software available on internet web sites such as http://blast.ncbi.nlm.nih.gov/ or http://www.ebi.ac.uk/Tools/emboss/). Those skilled in the art can determine appropriate parameters for measuring alignment, including any algorithms needed to achieve maximal alignment over the full length of the sequences being compared. For purposes herein, % nucleic acid sequence identity values refers to values generated using the pair wise sequence alignment program EMBOSS Needle that creates an optimal global alignment of two sequences using the Needleman-Wunsch algorithm, wherein all search parameters are set to default values, i.e. Scoring matrix = BLOSUM62, Gap open = 10, Gap extend = 0.5, End gap penalty = false, End gap open = 10 and End gap extend = 0.5.

As used herein, said nucleic acid construct comprises a transgene encoding MDR3 isoform A according to the invention and one or more control sequence required for expression of said coding sequence. Generally, the nucleic acid construct comprises a coding sequence and regulatory sequences preceding (5' non-coding sequences) and following (3' non-coding sequences) the coding sequence that are required for expression of the selected gene product. Thus, a nucleic acid construct typically comprises a promoter sequence, a coding sequence and a 3' untranslated region that usually contains a polyadenylation site and/or transcription terminator. The nucleic acid construct may also comprise additional regulatory elements such as, for example, enhancer sequences, a polylinker sequence facilitating the insertion of a DNA fragment within a vector and/or splicing signal sequences.

In one embodiment, the nucleic acid construct comprises a promoter. Said promoter initiates transgene expression upon introduction into a host cell. As used herein, the term "promoter" refers to a regulatory element that directs the transcription of a nucleic acid to which it is operably linked. A promoter can regulate both rate and efficiency of transcription of an operably linked nucleic acid. A promoter may also be operably linked to other regulatory elements which enhance ("enhancers") or repress ("repressors") promoter-dependent transcription of a nucleic acid. These regulatory elements include, without limitation, transcription factor binding sites, repressor and activator protein binding sites, and any other sequences of nucleotides known to one of skill in the art to act directly or indirectly to regulate the amount of transcription from the promoter, including e.g. attenuators, enhancers, and silencers. The promoter is located near the transcription start site of the gene or coding sequence to which is operably linked, on the same strand and upstream of the DNA sequence (towards the 5' region of the sense strand). A promoter can be about 100-1000 base pairs long. Positions in a promoter are designated relative to the transcriptional start site for a particular gene (i.e., positions upstream are negative numbers counting back from -1, for example -100 is a position 100 base pairs upstream).

As used herein, the term "operably linked" refers to a linkage of polynucleotide (or polypeptide) elements in a functional relationship. A nucleic acid is "operably linked" when it is placed into a functional relationship with another nucleic acid sequence. For instance, a promoter or transcription regulatory sequence is operably linked to a coding sequence if it affects the transcription of the coding sequence. Operably linked means that the DNA sequences being linked are typically contiguous; where it is necessary to join two protein encoding regions, they are contiguous and in reading frame.

In a particular embodiment, the nucleic acid construct of the invention further comprises a liver-specific promoter operably-linked to the transgene of the invention. In the context of this invention, a "liver-specific promoter" is a promoter which is more active in the liver than in any other tissue of the body. Typically, the activity of a liver specific promoter will be considerably greater in the liver than in other tissues. For example, such a promoter may be at least 2, at least 3, at least 4, at least 5 or at least 10 times more active (for example as determined by its ability to drive the expression in a given tissue in comparison to its ability to drive the expression in other cells or tissues). Accordingly, a liver specific promoter allows an active expression in the liver of the gene linked to it and prevents its expression in other cells or tissues.

In one embodiment, the liver-specific promoter is a nucleotide sequence of the α1-antitrypsin gene promoter (AAT or A1AT) (SEQ ID NO: 6), a bile salt-inducible promoter (SEQ ID NO: 7 or 8) or a chimeric promoter sequence EalbPa1AT that comprises a α1-antitrypsin gene promoter sequence (AAT or Pa1AT) combined with an albumin gene enhancer element (Ealb). All these promoter sequences have properties of liver specific promoters.

Each of these nucleic acid construct embodiments may also include a polyadenylation signal sequence; together or not with other optional nucleotide elements. As used herein, the term "polyadenylation signal" or "poly(A) signal" refers to a specific recognition sequence within 3' untranslated region (3' UTR) of the gene, which is transcribed into precursor mRNA molecule and guides the termination of the gene transcription. Poly(A) signal acts as a signal for the endonucleolytic cleavage of the newly formed precursor mRNA at its 3'-end, and for the addition to this 3'-end of a RNA stretch consisting only of adenine bases (polyadenylation process; poly(A) tail). Poly(A) tail is important for the nuclear export, translation, and stability of mRNA. In the context of the invention, the polyadenylation signal is a recognition sequence that can direct polyadenylation of mammalian genes and/or viral genes, in mammalian cells.

Poly(A) signals typically consist of a) a consensus sequence AAUAAA, which has been shown to be required for both 3'-end cleavage and polyadenylation of premessenger RNA (pre-mRNA) as well as to promote downstream transcriptional termination, and b) additional elements upstream and downstream of AAUAAA that control the efficiency of utilization of AAUAAA as a poly(A) signal. There is considerable variability in these motifs in mammalian genes.

In one embodiment, optionally in combination with one or more features of the various embodiments described above or below, the polyadenylation signal sequence of the nucleic acid construct of the invention is a polyadenylation signal sequence of a mammalian gene or a viral gene. Suitable polyadenylation signals include, among others, a SV40 early polyadenylation signal, a SV40 late polyadenylation signal, a HSV thymidine kinase polyadenylation signal, a protamine gene polyadenylation signal, an adenovirus 5 EIb polyadenylation signal, a growth hormone polydenylation signal, a PBGD polyadenylation signal, in silico designed polyadenylation signal (synthetic) and the like.

In a particular embodiment, the polyadenylation signal sequence of the nucleic acid construct is a synthetic poly(A) signal sequence based on the rabbit beta-globin gene, more particularly a synthetic poly(A) having sequence SEQ ID NO: 3.

### Expression vector

The nucleic acid construct of the invention may be comprised in an expression vector. As used herein, the term "expression vector" refers to a nucleic acid molecule used as a vehicle to transfer genetic material, and in particular to deliver a nucleic acid into a host cell, either *in vitro* or *in vivo.* Expression vector also refers to a nucleic acid molecule capable of effecting expression of a gene (transgene) in host cells or host organisms compatible with such sequences. Expression vectors typically include at least suitable transcription regulatory sequences and optionally, 3' transcription termination signals. Additional factors necessary or helpful in effecting expression may also be present, such as expression enhancer elements able to respond to a precise inductive signal (endogenous or chimeric transcription factors) or specific for certain cells, organs or tissues. Vectors include, but are not limited to, plasmids, phasmids, cosmids, transposable elements, viruses, and artificial chromosomes (e.g., YACs). Preferably, the vector of the invention is a vector suitable for use in gene or cell therapy, and in particular is suitable to target liver cells.

In some embodiments, the expression vector is a viral vector, such as vectors derived from Moloney murine leukemia virus vectors (MoMLV), MSCV, SFFV, MPSV or SNV, lentiviral vectors (e.g. derived from human immunodeficiency virus (HIV), simian immunodeficiency virus (SIV), feline immunodeficiency virus (FIV), bovine immunodeficiency virus (BIV) or equine infectious anemia virus (EIAV)), adenoviral (Ad) vectors, adeno-associated viral (AAV) vectors, simian virus 40 (SV-40) vectors, bovine papilloma virus vectors, Epstein-Barr virus, herpes virus vectors, vaccinia virus vectors, Harvey murine sarcoma virus vectors, murine mammary tumor virus vectors, Rous sarcoma virus vectors.

As is known in the art, depending on the specific viral vector considered for use, suitable sequences should be introduced in the vector of the invention for obtaining a functional viral vector, such as AAV ITRs for an AAV vector, or LTRs for lentiviral vectors. In a particular embodiment, said vector is an AAV vector.

AAV has arisen considerable interest as a potential vector for human gene therapy. Among the favourable properties of the virus are its lack of association with any human disease, its ability to infect both dividing and non-dividing cells, and the wide range of cell lines derived from different tissues that can be infected. The AAV genome is composed of a linear, single-stranded DNA molecule which contains 4681 bases (Berns and Bohenzky, 1987, Advances in Virus Research (Academic Press, Inc.) 32:243-307). The genome includes inverted terminal repeats (ITRs) at each end, which function in cis as origins of DNA replication and as packaging signals for the virus. The ITRs are approximately 145 bp in length. The internal non-repeated portion of the genome includes two large open reading frames, known as the AAV rep and cap genes, respectively. These genes code for the viral proteins involved in replication and packaging of the virion. In particular, at least four viral proteins are synthesized from the AAV rep gene, Rep 78, Rep 68, Rep 52 and Rep 40, named according to their apparent molecular weight. The AAV cap gene encodes at least three proteins, VP1, VP2 and VP3. For a detailed description of the AAV genome, see, e.g., Muzyczka, N. 1992 Current Topics in Microbiol. and Immunol. 158:97-129.

Thus, in one embodiment, the nucleic acid construct comprising transgene of the invention further comprises a 5'ITR and a 3'ITR sequences, preferably a 5'ITR and a 3' ITR sequences of an adeno-associated virus.

As used herein the term "inverted terminal repeat (ITR)" refers to a nucleotide sequence located at the 5'-end (5'ITR) and a nucleotide sequence located at the 3'-end (3'ITR) of a virus, that contain palindromic sequences and that can fold over to form T-shaped hairpin structures that function as primers during initiation of DNA replication. They are also needed for viral genome integration into the host genome; for the rescue from the host genome; and for the encapsidation of viral nucleic acid into mature virions. The ITRs are required in cis for the vector genome replication and its packaging into the viral particles.

AAV ITRs for use in the vectors of the invention may have a wild-type nucleotide sequence or may be altered by the insertion, deletion or substitution. The serotype of the inverted terminal repeats (ITRs) of the AAV vector may be selected from any known human or nonhuman AAV serotype. In specific embodiments, the expression viral vector may be carried out by using ITRs of any AAV serotype, including AAV1, AAV2, AAV3 (including types 3A and 3B), AAV4, AAV5, AAV6, AAV7, AAV8, AAV9, AAV10, AAV11, AAV12, avian AAV, bovine AAV, canine AAV, equine AAV, ovine AAV, and any other AAV serotype now known or later discovered.

In one embodiment, the nucleic acid construct further comprises a 5'ITR and a 3'ITR of an AAV of a serotype AAV2.

In a particular embodiment, the nucleic acid construct of the invention comprises or consists of SEQ ID NO: 4 or 5 or a sequence having at least 90% of identity with SEQ ID NO: 4 or 5.

In one embodiment, the nucleic acid construct or AAV vector genome according to the invention is comprised in a recombinant baculovirus genome. As used herein, the term "recombinant baculovirus genome" refers to a nucleic acid that comprises baculoviral genetic elements for autonomous replication of a recombinant baculovirus genome in a host cell permissive for baculovirus infection and replication, typically insect cells. The term "recombinant baculovirus genome" expressly includes genomes comprising nucleic acids that are heterologous to the baculovirus. Likewise, the term "recombinant baculovirus genome" does not necessarily refer to a complete baculovirus genome as the genome may lack viral sequences that are not necessary for completion of an infection cycle. In particular, the recombinant baculovirus genomes may include the heterologous AAV genes useful for rAAV production and/or the transgene such as codon-optimized MDR3 isoform A to be encapsidated in the rAAV for use in gene therapy. The baculoviral genetic elements for use in the present disclosure are preferably obtained from AcMNPV baculovirus (*Autographa californica* multinucleocapsid nucleopolyhedrovirus).

In a particular embodiment, the genes encoding baculovirus cathepsin and chitinase in said first and second baculoviral genomes are disrupted or deleted. In particular, the genes *v-cath* (Ac127) and *chiA* (Ac126) of the AcMNPV baculovirus may be disrupted or deleted so that the corresponding cathepsin or chitinase are either not expressed or expressed as inactive forms (i.e. have no enzymatic cathepsin or chitinase activity). In a particular embodiment, said recombinant baculovirus genomes are further disrupted or deleted for at least p24 gene (Ac129), preferably for the three baculoviral genes p10 (Ac137), p24 and p26 (Ac136). In a particular embodiment, said recombinant baculovirus genomes include functional p74 baculoviral gene (Ac138) (i.e. said gene has not been deleted or disrupted).

On the other hand, the nucleic acid construct or expression vector of the invention can be carried out by using synthetic 5'ITR and/or 3'ITR; and also by using a 5'ITR and a 3'ITR which come from viruses of different serotypes. All other viral genes required for viral vector replication can be provided in *trans* within the virus-producing cells (packaging cells) as described below. Therefore, their inclusion in the viral vector is optional.

In one embodiment, the nucleic acid construct or viral vector of the invention comprises a 5'ITR, a ψ packaging signal, and a 3'ITR of a virus. "ψ packaging signal" is a cis-acting nucleotide sequence of the virus genome, which in some viruses (e.g. adenoviruses, lentiviruses ...) is essential for the process of packaging the virus genome into the viral capsid during replication.

The construction of recombinant AAV viral particles is generally known in the art and has been described for instance in US 5,173,414 and US5,139,941; WO 92/01070, WO 93/03769, Lebkowski et al. (1988) Molec. Cell. Biol. 8:3988-3996; Vincent et al. (1990) Vaccines 90 (Cold Spring Harbor Laboratory Press); Carter, B. J. (1992) Current Opinion in Biotechnology 3:533-539; Muzyczka, N. (1992) Current Topics in Microbiol. and Immunol. 158:97-129; and Kotin, R. M. (1994) Human Gene Therapy 5:793-801.

### Viral particle

The nucleic acid construct or the expression vector of the invention may be packaged into a virus capsid to generate a "viral particle", also named "viral vector particle". In a particular embodiment, the nucleic acid construct or the expression vector is packaged into an AAV-derived capsid to generate an "adeno-associated viral particle" or "AAV particle". The present invention relates to a viral particle comprising a nucleic acid construct or an expression vector of the invention and preferably comprising capsid proteins of adeno-associated virus.

The term AAV vector particle encompasses any recombinant AAV vector particle or mutant AAV vector particle, genetically engineered. A recombinant AAV particle may be prepared by encapsidating the nucleic acid construct or viral expression vector including ITR(s) derived from a particular AAV serotype on a viral particle formed by natural or mutant Cap proteins corresponding to an AAV of the same or different serotype.

Proteins of the viral capsid of an adeno-associated virus include the capsid proteins VP1, VP2, and VP3. Differences among the capsid protein sequences of the various AAV serotypes result in the use of different cell surface receptors for cell entry. In combination with alternative intracellular processing pathways, this gives rise to distinct tissue tropisms for each AAV serotype. Several techniques have been developed to modify and improve the structural and functional properties of naturally occurring AAV viral particles (Bünning H et al. J Gene Med, 2008; 10: 717-733; Paulk et al. Mol ther. 2018.26(1):289-303; Wang L et al. Mol Ther. 2015. 23(12):1877-87; Vercauteren et al. Mol Ther. 2016.24(6):1042-1049; Zinn E et al., Cell Rep. 2015;12(6):1056-68).

Thus, in AAV viral particle according to the present disclosure, the nucleic acid construct or viral expression vector including ITR(s) of a given AAV serotype can be packaged, for example, into: a) a viral particle constituted of capsid proteins derived from the same or different AAV serotype [e.g. AAV2 ITRs and AAV5 capsid proteins; AAV2 ITRs and AAV8 capsid proteins; AAV2 ITRs and Anc80 capsid proteins; AAV2 ITRs and AAV9 capsid proteins]; b) a mosaic viral particle constituted of a mixture of capsid proteins from different AAV serotypes or mutants [e.g. AAV2 ITRs with AAV1 and AAV5 capsid proteins]; c) a chimeric viral particle constituted of capsid proteins that have been truncated by domain swapping between different AAV serotypes or variants [e.g. AAV2 ITRs with AAV5 capsid proteins with AAV3 domains].

The skilled person will appreciate that the AAV viral particle for use according to the present disclosure may comprise capsid proteins from any AAV serotype including AAV1, AAV2, AAV3 (including types 3A and 3B), AAV4, AAV5, AAV6, AAV7, AAV8, AAV9, AAV10, AAV11, AAV12, avian AAV, bovine AAV, canine AAV, equine AAV, ovine AAV, synthetic AAV variants such as NP40, NP59, NP84 (Paulk et al. Mol ther. 2018.26(1):289-303), LK03 (Wang L et al. Mol Ther. 2015. 23(12):1877-87), AAV3-ST (Vercauteren et al. Mol Ther. 2016.24(6):1042-1049), Anc80 (Zinn E et al., Cell Rep. 2015;12(6):1056-68) and any other AAV serotype now known or later discovered

In a specific embodiment, the AAV viral particle comprises capsid proteins from a serotype selected from the group consisting of an AAV1, AAV3B, an AAV5, an AAV7, an AAV8, and an AAV9 which are more suitable for delivery to the liver cells (Nathwani et al. Blood 2007; 109: 1414-1421; Kitajima et al. Atherosclerosis 2006; 186:65-73).

In a particular embodiment, the AAV viral particle comprises capsid proteins from Anc80, a predicted ancestor of viral AAVs serotypes 1, 2, 8, and 9 that behaves as a highly potent gene therapy vector for targeting liver, muscle and retina (Zinn E et al., Cell Report. 2015;12(6):1056-68). In a more particular embodiment, the viral particle comprises the Anc80L65 VP3 capsid protein (Genbank accession number: KT235804).

Thus, in a further aspect, the present invention relates to a viral particle comprising a nucleic acid construct or expression vector of the invention and preferably comprising capsid proteins of adeno-associated virus such as capsid proteins from Anc80 and AAV8 serotype, more preferably AAV8 serotype.

In a particular embodiment, the viral particle comprises AAV vector genome comprised in recombinant baculovirus. Thus, a second recombinant baculovirus genome comprising AAV rep and cap is used for producing AAV viral particle. In a particular embodiment, the rep and cap proteins are expressed from distinct baculovirus late promoters, preferably in inverse orientation. In a specific embodiment, that may be combined with the previous embodiments, the second baculovirus genome include a heterologous nucleic acid encoding the rep proteins, for example, rep proteins from AAV2 under the transcriptional control of the baculovirus polyhedron (P_{Ph}) promoter. In other embodiment, the second baculovirus genome includes a heterologous nucleic acid encoding the cap proteins under the transcriptional control of the p10 baculovirus promoter. Other modifications of the wild-type AAV sequences for proper expression in insect cells and/or to increase yield of VP and virion or to alter tropism or reduce antigenicity of the virion are also known in the art. By using helper baculoviral construct encoding the rep ORF (open reading frame) of an AAV serotype and cap ORF of a different serotype AAV, it is feasible packaging a vector flanked by ITRs of a given AAV serotype into virions assembled from structural capsid proteins of a different serotype. It is also possible by this same procedure to package mosaic, chimeric or targeted vectors. Virus-glycan interactions are critical determinants of host cell invasion. In a particular embodiment, the AAV viral particle comprises capsid proteins comprising one or more amino acids substitutions, wherein the substitutions introduce a new glycan binding site into the AAV capsid protein. In a more particular embodiment, the amino acid substitutions are in amino acid 266, amino acids 463-475 and amino acids 499-502 in AAV2 or the corresponding amino acid positions in AAV1, AAV3, AAV4, AAV5, AAV6, AAV7, AAV 8, AAV9, AAV10 or any other AAV serotype, also included Anc80 and Anc80L65.

The introduced new glycan binding site can be a hexose binding site [e.g. a galactose (Gal), a mannose (Man), a glucose (Glu) or a fucose (fuc) binding site]; a sialic acid (Sia) binding site [e.g. a Sia residue such as is N-acetylneuraminic acid (NeuSAc) or N-Glycolylneuraminic acid (NeuSGc)]; or a disaccharide binding site, wherein the disaccharide is a sialic acid linked to galactose, for instance in the form of Sia(alpha2,3)Gal or Sia(alpha2,6)Gal. Detailed guidance to introduce a new binding site from an AAV serotype into a capsid protein of an AAV of another serotype is given on international patent publication WO2014144229 and in Shen et al. (J. Biol. Chem. 2013; 288(40):28814-28823). In a particular embodiment, the Gal binding site from AAV9 is introduced into the AAV2 VP3 backbone resulting in a dual glycan-binding AAV strain which is able to use both HS and Gal receptors for cell entry. Preferably, said dual glycan-binding AAV strain is AAV2G9. Shen et al. generated AAV2G9 by substituting amino acid residues directly involved and immediately flanking the Gal recognition site on the AAV9 VP3 capsid protein subunit onto corresponding residues on the AAV2 VP3 subunit coding region (AAV2 VP3 numbering Q464V, A467P, D469N, I470M, R471A, D472V, S474G, Y500F, and S501A).

In another embodiment, the viral particle for use according to the present disclosure may be an adenoviral particle, such as an Ad5 viral particle. As it is the case for AAV viral particle, capsid proteins of Ad viral particles can also be engineered to modify their tropism and cellular targeting properties, alternative adenoviral serotypes can also be employed.

### A process for producing viral particles

Production of viral particles carrying the expression viral vector as disclosed above can be performed by means of conventional methods and protocols, which are selected taking into account the structural features chosen for the actual embodiment of expression vector and viral particle of the vector to be produced.

Briefly, viral particles can be produced in a host cell, more particularly in specific virus-producing cell (packaging cell), which is transfected with the nucleic acid construct or expression vector to be packaged, in the presence of a helper vector or virus or other DNA construct(s).

The term "packaging cells" as used herein, refers to a cell or cell line which may be transfected with a nucleic acid construct or expression vector of the invention, and provides in *trans* all the missing functions which are required for the complete replication and packaging of a viral vector. Typically, the packaging cells express in a constitutive or inducible manner one or more of said missing viral functions. Said packaging cells can be adherent or suspension cells.

Typically, a process of producing viral particles comprises the following steps:
a) culturing a packaging cell comprising a nucleic acid construct or expression vector as described above in a culture medium; and
b) harvesting the viral particles from the cell culture supernatant and/or inside the cells.

Conventional methods can be used to produce viral particles of the AAV viral particles, which consist on transient cell co-transfection with nucleic acid construct or expression vector (e.g. a plasmid) carrying the transgene of the invention; a nucleic acid construct (e.g., an AAV helper plasmid) that encodes rep and cap genes, but does not carry ITR sequences; and with a third nucleic acid construct (e.g., a plasmid) providing the adenoviral functions necessary for AAV replication. Viral genes necessary for AAV replication are referred herein as viral helper genes. Typically, said genes necessary for AAV replication are adenoviral helper genes, such as E1A, E1B, E2a, E4, or VA RNAs. Preferably, the adenoviral helper genes are of the Ad5 or Ad2 serotype.

Large-scale production of AAV particles according to the disclosure can also be carried out for example by infection of insect cells with a combination of recombinant baculoviruses (Urabe et al. Hum. Gene Ther. 2002; 13: 1935-1943). SF9 cells are co-infected with two or three baculovirus vectors respectively expressing AAV rep, AAV cap and the AAV vector to be packaged. The recombinant baculovirus vectors will provide the viral helper gene functions required for virus replication and/or packaging. Smith et al 2009 (Molecular Therapy, vol.17, no.11, pp 1888-1896) further describes a dual baculovirus expression system for large-scale production of AAV particles in insect cells.

Suitable culture media will be known to a person skilled in the art. The ingredients that compose such media may vary depending on the type of cell to be cultured. In addition to nutrient composition, osmolarity and pH are considered important parameters of culture media. The cell growth medium comprises a number of ingredients well known by the person skilled in the art, including amino acids, vitamins, organic and inorganic salts, sources of carbohydrate, lipids, trace elements (CuS04, FeS04, Fe(N03)3, ZnS04...), each ingredient being present in an amount which supports the cultivation of a cell *in vitro* (i.e., survival and growth of cells). Ingredients may also include different auxiliary substances, such as buffer substances (like sodium bicarbonate, Hepes, Tris...), oxidation stabilizers, stabilizers to counteract mechanical stress, protease inhibitors, animal growth factors, plant hydrolyzates, anti-clumping agents, anti-foaming agents. Characteristics and compositions of the cell growth media vary depending on the particular cellular requirements. Examples of commercially available cell growth media are: MEM (Minimum Essential Medium), BME (Basal Medium Eagle) DMEM (Dulbecco's modified Eagle's Medium), Iscoves DMEM (Iscove's modification of Dulbecco's Medium), GMEM, RPMI 1640, Leibovitz L-15, McCoy's, Medium 199, Ham (Ham's Media) F10 and derivatives, Ham F12, DMEM/F12, etc.

Further guidance for the construction and production of viral vectors for use according to the disclosure can be found in Viral Vectors for Gene Therapy, Methods and Protocols. Series: Methods in Molecular Biology, Vol. 737. Merten and Al-Rubeai (Eds.); 2011 Humana Press (Springer); Gene Therapy. M. Giacca. 2010 Springer-Verlag ; Heilbronn R. and Weger S. Viral Vectors for Gene Transfer: Current Status of Gene Therapeutics. In: Drug Delivery, Handbook of Experimental Pharmacology 197; M. Schäfer-Korting (Ed.). 2010 Springer-Verlag; pp. 143-170; Adeno-Associated Virus: Methods and Protocols. R.O. Snyder and P. Moulllier (Eds). 2011 Humana Press (Springer); Bünning H. et al. Recent developments in adeno-associated virus technology. J. Gene Med. 2008; 10:717-733; Adenovirus: Methods and Protocols. M. Chillón and A. Bosch (Eds.); Third Edition. 2014 Humana Press (Springer)

### Host cells

In another aspect, the invention relates to a host cell comprising a nucleic acid construct or an expression vector of the invention. More particularly, host cell according to the invention is a specific virus-producing cell, also named packaging cell which is transfected with the a nucleic acid construct or an expression vector according to the invention, in the presence of a helper vector or virus or other DNA constructs and provides in *trans* all the missing functions which are required for the complete replication and packaging of a viral particle. Said packaging cells can be adherent or suspension cells

For example, said packaging cells may be eukaryotic cells such as mammalian cells, including simian, human, dog and rodent cells. Examples of human cells are PER.C6 cells (WO01/38362), MRC-5 (ATCC CCL-171), WI-38 (ATCC CCL-75), HEK-293 cells (ATCC CRL-1573), HeLa cells (ATCC CCL2) and fetal rhesus lung cells (ATCC CL- 160). Examples of non-human primate cells are Vero cells (ATCC CCL81), COS-1 cells (ATCC CRL-1650) or COS-7 cells (ATCC CRL-1651). Examples of dog cells are MDCK cells (ATCC CCL-34). Examples of rodent cells are hamster cells, such as BHK21-F, HKCC cells, or CHO cells.

As an alternative to mammalian sources, the packaging cells for producing the viral particles may be derived from avian sources such as chicken, duck, goose, quail or pheasant. Examples of avian cell lines include avian embryonic stem cells (WO01/85938 and WO03/076601), immortalized duck retina cells (WO2005/042728), and avian embryonic stem cell derived cells, including chicken cells (WO2006/108846) or duck cells, such as EB66 cell line (WO2008/129058 & WO2008/142124).

In another embodiment, the cells can be any cells permissive for baculovirus infection and replication packaging cells. In a particular embodiment, said cells are insect cells, such as SF9 cells (ATCC CRL-1711), Sf21 cells (IPLB-Sf21), MG1 cells (BTI-TN-MG1) or High Five^{™} cells (BTI-TN-5B1-4).

Accordingly, in a particular embodiment, optionally in combination with one or more features of the various embodiments described above or below, the host cell comprises:
- A nucleic acid construct or expression vector comprising a transgene encoding MDR3 isoform A according to the invention (e.g., the AAV vector according to the invention),
- a nucleic acid construct, for example a plasmid, encoding AAV rep and/or cap genes which does not carry the ITR sequences; and/or
- a nucleic acid construct, for example a plasmid or virus, comprising viral helper genes.

In another aspect, the invention relates to a host cell transduced with a viral particle of the invention and the term "host cell" as used herein refers to any cell line that is susceptible to infection by a virus of interest, and amenable to culture *in vitro.*

The host cell of the invention may be used for *ex vivo* gene therapy purposes. In such embodiments, the cells are transduced with the viral particle of the invention and subsequently transplanted to the patient or subject. Transplanted cells can have an autologous, allogenic or heterologous origin. For clinical use, cell isolation will generally be carried out under Good Manufacturing Practices (GMP) conditions. Before transplantation, cell quality and absence of microbial or other contaminants is typically checked and liver preconditioning, such as with radiation and/or an immunosuppressive treatment, may be carried out. Furthermore, the host cells may be transplanted together with growth factors to stimulate cell proliferation and/or differentiation, such as Hepatocyte Growth Factor (HGF).

In a particular embodiment, the host cell is used for *ex vivo* gene therapy into the liver. Preferably, said cells are eukaryotic cells such as mammalian cells, these include, but are not limited to, humans, non-human primates such as apes; chimpanzees; monkeys, and orangutans, domesticated animals, including dogs and cats, as well as livestock such as horses, cattle, pigs, sheep, and goats, or other mammalian species including, without limitation, mice, rats, guinea pigs, rabbits, hamsters, and the like. A person skilled in the art will choose the more appropriate cells according to the patient or subject to be transplanted.

Said host cell may be a cell with self-renewal and pluripotency properties, such as stem cells or induced pluripotent stem cells. Stem cells are preferably mesenchymal stem cells. Mesenchymal stem cells (MSCs) are capable of differentiating into at least one of an osteoblast, a chondrocyte, an adipocyte, or a myocyte and may be isolated from any type of tissue. Generally MSCs will be isolated from bone marrow, adipose tissue, umbilical cord, or peripheral blood. Methods for obtaining thereof are well known to a person skilled in the art. Induced pluripotent stem cells (also known as iPS cells or iPSCs) are a type of pluripotent stem cell that can be generated directly from adult cells. Yamanaka et al. induced iPS cells by transferring the Oct3/4, Sox2, Klf4 and c-Myc genes into mouse and human fibroblasts, and forcing the cells to express the genes (WO 2007/069666). Thomson et al. subsequently produced human iPS cells using Nanog and Lin28 in place of Klf4 and c-Myc (WO 2008/118820).

Said host cells may also be hepatocytes. Hepatocyte transplantation procedures, including cell isolation and subsequent transplantation into a human or mice recipient is described for instance in Filippi and Dhawan, Ann NY Acad Sci. 2014, 1315 50-55; Yoshida et al., Gastroenterology 1996, 111: 1654-1660; Irani et al. Molecular Therapy 2001, 3:3, 302-309; and Vogel et al. J Inherit Metab Dis 2014, 37:165-176. A method for ex vivo transduction of a viral vector into hepatocytes is described for instance in Merle et al., Scandinavian Journal of Gastroenterology 2006, 41:8, 974-982.

### Pharmaceutical compositions

Another aspect of the present disclosure relates to a pharmaceutical composition comprising a nucleic acid construct, an expression vector, a viral particle or a host cell of the invention in combination with one or more pharmaceutical acceptable excipient, diluent or carrier.

As used herein, the term "pharmaceutically acceptable" means approved by a regulatory agency or recognized pharmacopeia such as European Pharmacopeia, for use in animals and/or humans. The term "excipient" refers to a diluent, adjuvant, carrier, or vehicle with which the therapeutic agent is administered.

Any suitable pharmaceutically acceptable carrier, diluent or excipient can be used in the preparation of a pharmaceutical composition (See e.g., Remington: The Science and Practice of Pharmacy, Alfonso R. Gennaro (Editor) Mack Publishing Company, April 1997). Pharmaceutical compositions are typically sterile and stable under the conditions of manufacture and storage. Pharmaceutical compositions may be formulated as solutions (e.g. saline, dextrose solution, or buffered solution, or other pharmaceutically acceptable sterile fluids), microemulsions, liposomes, or other ordered structure suitable to accommodate a high product concentration (e.g. microparticles or nanoparticles). The carrier may be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition.

Prolonged absorption of the injectable compositions can be brought about by including in the composition an agent which delays absorption, for example, monostearate salts and gelatin. The product of the invention may be administered in a controlled release formulation, for example in a composition which includes a slow release polymer or other carriers that protect the product against rapid release, including implants and microencapsulated delivery systems. Biodegradable and biocompatible polymers may for example be used, such as ethylene vinyl acetate, polyanhydrides, polyglycolic acid, collagen, polyorthoesters, polylactic acid and polylactic / polyglycolic copolymers (PLG). Preferably, said pharmaceutical composition is formulated as a solution, more preferably as an optionally buffered saline solution. Supplementary active compounds can also be incorporated into the pharmaceutical compositions of the invention. Guidance on co-administration of additional therapeutics can for example be found in the Compendium of Pharmaceutical and Specialties (CPS) of the Canadian Pharmacists Association.

In one embodiment, the pharmaceutical composition is a parenteral pharmaceutical composition, including a composition suitable for intravenous, intraarterial, subcutaneous, intraperitoneal or intramuscular administration. These pharmaceutical compositions are exemplary only and do not limit the pharmaceutical compositions suitable for other parenteral and non-parenteral administration routes. The pharmaceutical compositions described herein can be packaged in single unit dosage or in multidosage forms.

### Therapeutic uses

In a further aspect, the invention relates to a nucleic acid construct, expression vector, viral particle, host cell or pharmaceutical composition of the invention for use as a medicament in a subject in need thereof.

The term "subject" or "patient" as used herein, refers to mammals. Mammalian species that can benefit from the disclosed methods of treatment include, but are not limited to, humans, non-human primates such as apes, chimpanzees, monkeys, and orangutans, domesticated animals, including dogs and cats, as well as livestock such as horses, cattle, pigs, sheep, and goats, or other mammalian species including, without limitation, mice, rats, guinea pigs, rabbits, hamsters, and the like. In particular embodiment, said subject is neonate, an infant or, a child, more particularly a neonate or an infant. As used herein "neonate" refers to a baby who is less than 28 days and "infants" as used herein refers to a child who is between 29 days and 2 years.

In an additional aspect, the invention relates to a nucleic acid construct, expression vector, viral particle, host cell or pharmaceutical composition of the invention for use in the treatment of a liver disease, in particular familiar cholestasis type 3 (PFIC3) in a subject in need thereof. As used herein, the term "treatment", "treat" or "treating" refers to any act intended to ameliorate the health status of patients such as therapy, prevention, prophylaxis and retardation of the disease. In certain embodiments, such term refers to the amelioration or eradication of a disease or symptoms associated with a disease. In other embodiments, this term refers to minimizing the spread or worsening of the disease resulting from the administration of one or more therapeutic agents to a subject with such a disease.

In a particular embodiment, liver disease is selected from the group consisting of: intrahepatic cholestasis of pregnancy type 3 (ICP3), cholesterol gallstone disease, drug-induced cholestasis, transient neonatal cholestasis, adult idiopathic cirrhosis, cholangiocarcinoma, and familiar cholestasis type 3 (PFIC3). In a more particular embodiment, said liver disease is familiar cholestasis type 3 (PFIC3).

In a related aspect, the invention pertains to the use of a nucleic acid construct, expression vector, viral particle, host cell or pharmaceutical composition of the invention in the preparation of a medicament for use in the treatment of a liver disease, preferably for use in the treatment of familiar cholestasis type 3 (PFIC3).

Described herein but not part of the invention is a method of treating and/or preventing a liver disease, preferably familiar cholestasis type 3 (PFIC3), in a subject in need thereof that comprises administering to the subject a therapeutically effective amount of a nucleic acid construct, expression vector, a viral particle, a host cell or a pharmaceutical composition of the invention.

In the context of the invention, an "effective amount" means a therapeutically effective amount. As used herein a "therapeutically effective amount" refers to an amount effective, at dosages and for periods of time necessary to achieve the desired therapeutic result, such as decreases in biomarkers levels in serum such as gamma-glutamyltransferase (GGT), alanine transaminase (ALT), alkaline phosphatase (ALP), aspartate transaminase (AST), bile salts (BS) and bilirubin, restoration of bile phosphatidylcholine or other phospholipid concentrations. The therapeutically effective amount of the product of the invention, or pharmaceutical composition that comprises it may vary according to factors such as the disease state, age, sex, and weight of the individual, and the ability of the product or pharmaceutical composition to elicit a desired response in the individual. Dosage regimens may be adjusted to provide the optimum therapeutic response. A therapeutically effective amount is also typically one in which any toxic or detrimental effect of the product or pharmaceutical composition is outweighed by the therapeutically beneficial effects.

The treatment with a product of the invention may alleviate, ameliorate, or reduce the severity of one or more symptoms of familiar cholestasis type 3 (PFIC3). For example, treatment may decrease biomarker levels in serum such as gamma-glutamyltransferase (GGT) alanine transaminase (ALT), alkaline phosphatase (ALP), aspartate transaminase (AST), bile salts (BS) and bilirubin; or restore bile phosphatidylcholine or other phospholipid concentrations; and as a consequence may alleviate, ameliorate, or reduce the severity of cholangitis, ductular proliferation, biliary fibrosis, portal hypertension, cirrhosis, gastrointestinal bleeding, icterus, cholestasis, pruritus, and liver failure.

The product of the invention will be typically included in a pharmaceutical composition or medicament, optionally in combination with a pharmaceutical carrier, diluent and/or adjuvant. Such composition or medicinal product comprises the product of the invention in an effective amount, sufficient to provide a desired therapeutic effect, and a pharmaceutically acceptable carrier or excipient.

In one embodiment the nucleic acid construct, expression vector, the viral particle, the host cell or the pharmaceutical composition for its therapeutic use is administered to the subject or patient by a parenteral route, in particularly by intravenous, intraarterial, subcutaneous, intraperitoneal, or intramuscular route.

In one embodiment, the nucleic acid construct, expression vector, the viral particle, the host cell or the pharmaceutical composition for its therapeutic use is administered by interstitial route, i.e. by injection to or into the interstices of a tissue. The tissue target may be specific, for example the liver tissue, or it may be a combination of several tissues, for example the muscle and liver tissues. Exemplary tissue targets may include liver, skeletal muscle, heart muscle, adipose deposits, kidney, lung, vascular endothelium, epithelial and/or hematopoietic cells. In a preferred embodiment, it is administered by intrahepatic injection, i.e. injection into the interstitial space of hepatic tissue.

The amount of product of the invention that is administered to the subject or patient may vary depending on the particular circumstances of the individual subject or patient including, age, sex, and weight of the individual; the nature and stage of the disease, the aggressiveness of the disease; the route of administration; and/or concomitant medication that has been prescribed to the subject or patient. Dosage regimens may be adjusted to provide the optimum therapeutic response.

For any particular subject, specific dosage regimens may be adjusted over time according to the individual needs and the professional judgment of the person administering or supervising the administration of the compositions. Dosage ranges set forth herein are exemplary only and do not limit the dosage ranges that may be selected by medical practitioners.

In one embodiment, an AAV viral particle according to the invention can be administered to the subject or patient for the treatment of PFIC3 disease in an amount or dose comprised within a range of 5 × 10¹¹ to 1 × 10¹⁶ vg / kg (vg: viral genomes; kg: subject's or patient's body weight). In a more particular embodiment, the AAV viral particle is administered in an amount comprised within a range of 1 × 10¹³ to 1 × 10¹⁵ vg / kg. In a more particular embodiment, the AAV viral particle is administered at a dosage of at least 2 × 10¹³ vg / kg, preferably 4.5 × 10¹³ vg / kg, more preferably 5 × 10¹³ vg / kg, and more preferably 8 × 10¹³ VG / kg.

### Kit

In another aspect, the invention further relates to a kit comprising a nucleic acid construct, expression vector, a host cell, viral particle or pharmaceutical composition of the invention in one or more containers. The kit may include instructions or packaging materials that describe how to administer the nucleic acid construct, expression vector, viral particle, host cell or pharmaceutical compositions contained within the kit to a patient. Containers of the kit can be of any suitable material, e.g., glass, plastic, metal, etc., and of any suitable size, shape, or configuration. In certain embodiments, the kits may include one or more ampoules or syringes that contain the products of the invention in a suitable liquid or solution form.

The following examples are provided by way of illustration, and they are not intended to be limiting of the present invention. Furthermore, the present invention covers all possible combinations of particular and preferred embodiments described herein.

### EXAMPLES

### Six MDR3 transgene variants analyzed in vitro by transfection

The ABCB4 gene encodes for the multi-drug resistance protein (MDR3), a phosphatidylcholine (PC) transporter protein that localizes to the canalicular membrane of hepatocytes and is responsible for regulating PC concentration in the bile. PC forms mixed micelles with bile acids and serves to reduce tissue damage and toxicity caused by free bile acids. Mutations in ABCB4 can lead to a deficiency of PC in the bile causing toxicity, which leads to cholestasis. Progressive familiar intrahepatic cholestasis type 3 is the resulting disorder (Jacquemin, E. 2012, Clin Res Hepatol Gastroenterol; 36 Suppl 1:526-35). Three potential isoforms may exist for MDR3, designated A, B, and C. Plasmids containing either the wild-type (wt) or codon-optimized (co) version of human MDR3-A, -B or -C cDNA under the transcriptional control of the liver specific alpha 1-anti-trypsin (A1AT) promoter were generated. These plasmids were transiently transfected into the human hepatic cell line Huh7 in order to analyse and compare expression of the six variants. Forty-eight hours post-transfection, cells were harvested, fixed, stained with rabbit anti-MDR3 primary and anti-rabbit-Alexa488 secondary antibodies, and visualized by immunofluorescence and confocal microscopy. Images of cells that stained positive for MDR3 expression showed that only isoform A appeared to localize to the cell membrane. This was more apparent with confocal microscopy as serial planes of the MDR3-positive cells showed clear membrane-localized expression (Figures 1 and 2). Of note, sequences for potential isoforms B and C (identified in the case of C via gene prediction of alternate splicing variants based on expressed sequence tag (EST) data) vary from the A isoform due to the presence of a 7 amino acid addition near the nucleotide binding domain 2 and a deletion of 47 amino acids that include transmembrane domain eleven, respectively. There is no experimental confirmation of the natural existence of isoform C (UniProt entry P21439).

### Only codon optimized MDR3-A isoform is compatible with efficient AAV vector production

With data showing only isoform A localized to the membrane of Huh7 cells when expressed from a transiently transfected plasmid, AAV vectors based on the artificial Anc80 variant (Zinn E et al., Cell Rep.; 2015, 12(6):1056-68) encoding either the wildtype (MDR3-Awt) or the codon optimized (MDR3-Aco) transgene variant of MDR3 isoform A were produced. The most conventional AAV vector production process is based on transient transfection of HEK293 cell with a set of 2 or 3 plasmids providing all the functions necessary for vector genome amplification, capsid production and viral particle assembly. One of these plasmids contains the transgene flanked by two AAV inverted terminal repeats (ITR) and must be produced in sufficient quantities to allow production of the vector. AAV plasmids containing the MDR3-Awt and MDR3-Aco sequences downstream of the A1AT promoter were generated. Unexpectedly, the inventors encountered issues in growing bacteria harboring the MDR3-Awt AAV plasmid, such as reduced bacterial growth rate and higher instances of recombination as seen by abnormal restriction enzyme digestion analyses of the isolated plasmid DNA. These issues were partially remedied in plasmid production by reducing the growth temperature to 30°C but recombined/impure plasmid DNA contaminant remain unresolved. In contrast, the AAV plasmid containing the MDR3-Aco sequence was much more stable and could be produced without any of the above technical issues.

AAV vectors were produced with the MDR3 plasmids by transient transfection of HEK293 cells. Briefly, to produce AAVAnc80 viral particles, thirty 150cm²-flasks containing confluent HEK293T cell monolayers were co-transfected with helper plasmids pδF6, pAAP2, pKAnc80L65 (Zinn E et al., Cell Report; 2015, 12(6):1056-68) and the chosen AAV plasmid containing the vector sequence to be packaged (AAV-MDR3-Aco or AAV-MDR3-Awt) using polyethyenimine (PEI). After 72h of incubation, AAV particles were purified from the supernatant and cells by ultracentrifugation using an iodixanol gradient as described in Vanrell L. et al., Mol Ther. 2011;19(7):1245-53. Finally, the purified virus was concentrated using Amicon Ultra Centrifugal Filters-Ultracel 100K (Millipore) and titrated by quantitative PCR using oligonucleotides specific for the A1AT promoter (Forward primer: 5'-TTGCTCCTCCGATAACTGGG-3' (SEQ ID NO: 9); Reverse primer: 5'-CCCTGTCCTCGTCCGTATTT-3') (SEQ ID NO: 10). AAV8 viral particles were produced in the same way, but using pDP8 (PlasmidFactory, Germany) as a single helper plasmid. AAVAnc80-MDR3-Aco viral particles were routinely produced at quantities and concentration compatible with *in vivo* studies (Table 1) with an average titer of 4.6×10¹² viral genomes (VG)/mL. Surprisingly, 4 out of 5 attempts to produce the AAVAnc80 vectors with the MDR3-Awt isoform failed to achieve any significant yield (4 out of 5 had titers less than 5×10¹¹ VG/mL, which were too low for *in vivo* application). This indicated an unexpected intrinsic feature of the MDR3-Awt isoform coding sequence not only when carried as a plasmid, as observed previously, but also in the context of an AAV particle. It was estimated that production of AAV vectors harboring the MDR3-Awt version would require substantial troubleshooting and/or higher amounts of time, resources, and materials than production of the codon optimized MDR3-Aco. Therefore, isoform MDR3-Aco was selected for further development.

**Table 1: AAV virus production yields**

| AAV VECTOR | | VOLUME (µL) | TITER (VG/mL) |
|---|---|---|---|
| AAVAnc80-MDR3-Aco | AAVAnc80-MDR3-Aco#1 | 950 | 1.18E+12 |
| | AAVAnc80-MDR3-Aco#2 | 950 | 5.13E+12 |
| | AAVAnc80-MDR3-Aco#3 | 950 | 7.61E+12 |
| AAVAnc80-MDR3-Awt | AAVAnc80-MDR3-Awt#1 | 950 | 1.5E+12 |
| | AAVAnc80-MDR3-Awt#2 | 950 | 3.86E+10 |
| | AAVAnc80-MDR3-Awt#3 | 950 | 8.13E+10 |
| | AAVAnc80-MDR3-Awt#4 | 980 | 3.13E+11 |
| | AAVAnc80-MDR3-Awt#5 | 1050 | 1.66E+11 |

### In vivo testing of liver expression of MDR3 WT and CO variants via hydrodynamic injection (plasmid DNA transfection)

The transgenic mouse model FVB.129P2-Abcb4tm1Bor/J (Abcb4^{-/-}) is a homozygous knockout for the ABCB4 gene (Smit J.J. et al., Cell. 1993;75(3):451-62). *In vivo* MDR3 expression was analyzed in these mice following delivery of plasmids containing the MDR3 transgene variants via hydrodynamic injection (HDI). Seven-week old male Abcb4^{-/-} mice were injected into the tail vein with 25µg of plasmid pAAV-MDR3 in 2.4 mL total volume in under 5 seconds. Plasmids with MDR3 isoforms Aco, Awt, Cco, and Cwt were tested. The animals were sacrificed 24h later and livers were harvested and stained for MDR3 expression by immunohistochemistry (IHC). Both mice inoculated with pAAV-MDR3-Aco showed MDR3 expression. In particular, one mouse showed MDR3 expression in discreet pockets throughout the liver tissue (Figure 3) with MDR3 clearly located on the canalicular membrane of hepatocytes. In contrast, HDI with pAAV-MDR3-Awt resulted in only one animal showing MDR3 expression that could be detected around only one cell in the whole sample (Figure 3). These results suggest that the MDR3-Aco sequence can be expressed much more efficiently *in vivo* compared to MDR3-Awt. Finally, neither pAAV-MDR3-Cco nor pAAV-MDR3-Cwt showed any positive staining for MDR3 (data not shown).

### Testing of AAV vectors harboring MDR3 transgene in a mouse model for PFIC3

The Abcb4^{-/-} knock-out mouse model has shown to reliably replicate several PFIC3-associated markers, such as elevated serum liver transaminase, alkaline phosphatase, bile salt and bilirubin levels; increased liver and spleen size; decreased concentration of phosphatidylcholine in the bile; and severe morphological abnormalities in the liver, such as fibrosis, collagen deposits, and cell infiltrates. The onset of symptoms in these mice appears before 4 weeks of age. The inventors initially tested if these mice were susceptible to AAV infection using an AAV vector based on the Anc80 variant harboring the transgene for a green fluorescent protein (GFP) reporter.

Mice (at 3 or 4 weeks of age) inoculated with 5×10¹² VG/kg body weight were sacrificed one week later, and liver samples were processed for quantitation of AAV vector genome copies and GFP mRNA copies by qPCR and RT-qPCR, respectively. Neither 3- nor 4-week-old Abcb4^{-/-} mice were found to be transduced with the Anc80 vector at significant levels when compared to WT mice inoculated at the same ages (data not shown).

Next, 2- or 3-week-old Abcb4^{-/-} mice were injected with AAVAnc80 vector harboring the MDR3-Aco transgene at three different doses (5×10¹², 1.5×10¹³ and 5×10¹³ VG/kg). Mice treated at 3 weeks of age and sacrificed 2 weeks later showed only a very minimal improvement in PFIC3 symptoms (serum biomarker levels and spleen and liver size) only in males with the 5×10¹³ dose (data not shown), while a slight increase in bile PC concentration was observed in both males and females that received the highest AAV dose (p=0.057 vs. saline-treated Abcb4^{-/-} mice) (Figure 4).

However, mice treated at two weeks of age with AAVAnc80-MDR3-Aco at 5×10¹³ VG/kg showed marked improvements in serum biomarkers up through 3 weeks post-treatment (Figure 5). When these mice were sacrificed at 3 weeks post-treatment, a reversion was seen back towards wildtype characteristics in liver and spleen weight and liver histology (Figures 6 and 7). Mice treated with a 3-times lower dose (1.5×10¹³ VG/kg), however, showed little to no improvement over negative control saline-treated ABCB4^{-/-} mice. Bile PC concentration was also significantly increased in mice treated at the highest dose (Figure 8).

### AAV serotype 8 vectors achieve sustained therapeutic response in PFIC3 mice.

AAV vector of serotype 8 harboring the MDR3-Aco transgene were generated and tested in Abcb4^{-/-} mice. The AAV8-MDR3-Aco vector was administered into mice at two weeks of age with either 1.5×10¹³ or 5×10¹³ VG/kg dose. As early as 1-2 weeks post-treatment, the biomarker levels in serum (alanine transaminase (ALT), alkaline phosphatase (ALP), aspartate transaminase (AST), bile salts (BS) and bilirubin of mice treated with the highest dose clearly reverted away from levels shown in the diseased animals to wild-type animal levels (Figure 9). A reversion was seen back towards wildtype characteristics in liver and spleen weight and liver histology (Figure 10). This liver disease reversion was maintained for the entire duration of the study (up through 12 weeks post-treatment). Meanwhile, the biomarker levels of the lower dose-treated mice did not vary from those of saline-treated Abcb4^{-/-} mice.

### MDR3 expression achieved at high levels in PFIC3 mice treated with AAV8-MDR3-Aco.

MDR3 expression was analyzed by immunohistochemistry (IHC) with an anti-human MDR3 antibody in livers of Abcb4^{-/-} mice treated with AAV8-MDR3-Aco at 5×10¹³ VG/kg. Substantial MDR3 expression was detected in livers with a clear protein localization on the canalicular membranes of hepatocytes (Figure 11), which was comparable with the pattern and intensity of expression observed in wild-type mice (these mice stained under the same conditions provided a comparator since the synthetic peptide for generating the anti-hMDR3 had 100% sequence identity with the sequence of the mouse version of MDR3). Saline-treated control animals showed no expression of MDR3 as expected.

### AAV8-MDR3 treatment in PFIC3 mice exhibits a dose response.

Abcb4^{-/-} mice were treated with five different doses of AAV8-MDR3 (5×10¹², 1×10¹³, 2×10¹³, 4×10¹³, and 8×10¹³ VG/kg). After 3 weeks post-treatment, a clear dose-related response is observed for serum biomarker levels (Figure 12).

### AAV serotype 8 vectors achieve sustained therapeutic response in 5-week-old PFIC3 mice.

The AAV8-MDR3-Aco vector was administered to Abcb4^{-/-} mice at 5 weeks of age at 1×10¹⁴ VG/kg. Throughout a 12-week follow-up, biomarker levels reverted from diseased state down to levels seen in wild-type animals (Figure 13), as was seen when treated at 2 weeks of age.

The effect was more consistent in males than in females. At 17 weeks of age the mice were sacrificed and the AAV8-MDR3-Aco-treated mice showed clearly reduced evidence of PFIC3 disease, including reduced liver and spleen size (Figure 14 a-b), increased bile PC (Figure 14c) and reduced liver fibrosis (Figure 14d). Expression of MDR3 protein was measured via staining with immunohistochemistry using an antibody specific to MDR3. Expression levels were 66% of wild-type levels in males and 31% in females (Figure 14e). Bile PC was restored to 54% and 25% wild-type levels in males and females, respectively.

In conclusion, the inventors have found that a codon optimized version of the isoform A of MDR3 is the best candidate for developing a gene therapy vector to potentially treat PFIC3 patients. Only isoform A was found to localize to the cell membrane when expression was tested by DNA transfection in a human hepatic cell line *in vitro.* Surprisingly, only the codon optimized MDR3-A showed an efficient *in vivo* expression when administered as naked DNA.

When initiating production of AAV vectors (Anc80 and AAV8) harboring the MDR3-A transgene, only the codon optimized version was viable for vector production at expected standard yield.

From testing of these AAV vectors harboring the MDR3-Aco transgene in an Abcb4-/- KO mouse model, mice treated at 2 weeks of age showed high expression levels of MDR3 with localization to the canalicular membranes of hepatocytes and significant restoration of biomarker levels, achieving a therapeutic effect. In studies utilizing the ancestral serotype Anc80, vector treatment at 5×10¹³ VG/kg achieved reversion of PFIC3 serum biomarker levels (up through 3 weeks post-treatment), decreases in liver and spleen sizes, increase in bile PC, and a correction of the liver morphology abnormalities observed in cholestatic mice. At 5×10¹³ VG/kg of AAV8-MDR3-Aco, serum biomarker levels indicative of PFIC3 disease were reverted to the normal levels observed in wildtype animals up through 3 months post-treatment. Moreover, in liver and spleen size, bile PC, and liver morphology, these AAV8-MDR3-Aco-treated mice also demonstrated marked improvement in PFIC3 characteristics 3 months after being treated.

### Sequences for use in practicing the invention

Sequences for use in practicing the invention are described below:
**Codon-optimized sequence encoding MDR3 isoform A (co-MDR3(A)) : (SEQ ID NO: 1)** 
ATGGATCTGGAGGCCGCCAAGAAcGGCACCGCcTGGAGACCCACAAGCGCCGAGGGCGACTTCGAGCTGGGCATCAGCT
CCAAGCAGAAGAGAAAGAAGACCAAGACAGTGAAGATGATCGGCGTGCTGACACTGTTCAGGTACTCCGACTGGCAGGA
TAAGCTGTTTATGTCTCTGGGCACCATCATGGCAATCGCCCACGGCAGCGGCCTGCCTCTGATGATGATCGTGTTCGGCGA
GATGACCGACAAGTTTGTGGATACAGCCGGCAATTTCTCCTTTCCCGTGAACTTCTCTCTGAGCCTGCTGAACCCTGGCAAG
ATCCTGGAGGAGGAGATGACAAGATATGCCTACTATTACTCTGGCCTGGGAGCAGGCGTGCTGGTGGCAGCATACATCCA
GGTGAGCTTCTGGACCCTGGCAGCAGGCCGGCAGATCAGAAAGATCAGGCAGAAGTTCTTTCACGCCATCCTGCGCCAGG
AGATCGGCTGGTTTGACATCAATGATACCACAGAGCTGAACACCCGGCTGACAGACGACATCTCTAAGATCAGCGAGGGC
ATCGGCGATAAAGTGGGCATGTTCTTTCAGGCCGTGGCCACATTCTTTGCCGGCTTCATCGTGGGCTTTATCAGGGGCTGG
AAGCTGACCCTGGTCATCATGGCCATCTCTCCAATCCTGGGCCTGAGCGCCGCCGTGTGGGCAAAGATCCTGTCCGCCTTC
TCTGACAAGGAGCTGGCCGCCTACGCCAAGGCAGGAGCAGTGGCAGAGGAGGCCCTGGGCGCCATCCGCACCGTGATCG
CCTTTGGCGGCCAGAATAAGGAGCTGGAGCGGTATCAGAAGCACCTGGAGAACGCCAAGGAGATCGGCATCAAGAAGG
CCATCTCCGCCAATATCTCTATGGGCATCGCCTTCCTGCTGATCTATGCCAGCTACGCCCTGGCCTTTTGGTATGGCAGCAC
CCTGGTCATCAGCAAGGAGTACACCATCGGCAATGCCATGACAGTGTTCTTTAGCATCCTGATCGGAGCCTTCTCCGTGGG
ACAGGCAGCACCATGCATCGACGCCTTCGCCAACGCCAGAGGCGCAGCCTACGTGATCTTTGACATCATCGATAACAATCC
AAAGATCGACTCCTTTTCTGAGCGGGGCCACAAGCCCGATTCCATCAAGGGCAATCTGGAGTTCAACGACGTGCACTTTAG
CTATCCCTCCCGCGCCAATGTGAAGATCCTGAAGGGCCTGAACCTGAAGGTGCAGTCCGGACAGACAGTGGCCCTGGTGG
GCTCTAGCGGATGCGGCAAGTCTACCACAGTGCAGCTGATCCAGCGGCTGTATGACCCAGATGAGGGCACCATCAACATC
GACGGCCAGGACATCCGCAACTTCAATGTGAACTACCTGCGGGAGATCATCGGCGTGGTGTCTCAGGAGCCCGTGCTGTT
TAGCACCACAATCGCCGAGAATATCTGTTATGGCCGCGGCAACGTGACAATGGATGAGATCAAGAAGGCCGTGAAGGAG
GCCAATGCCTACGAGTTCATCATGAAGCTGCCCCAGAAGTTTGACACCCTGGTGGGAGAGAGAGGCGCCCAGCTGAGCG
GAGGCCAGAAGCAGAGGATCGCCATCGCCAGAGCCCTGGTGAGGAACCCTAAGATCCTGCTGCTGGACGAGGCCACCTC
CGCCCTGGATACAGAGTCTGAGGCAGAGGTGCAGGCCGCCCTGGATAAGGCCCGCGAGGGACGGACCACAATCGTGATC
GCCCACAGACTGAGCACAGTGAGGAATGCCGACGTGATCGCCGGCTTCGAGGATGGCGTGATCGTGGAGCAGGGCAGCC
ACTCCGAGCTGATGAAGAAGGAGGGCGTGTACTTCAAGCTGGTGAACATGCAGACCTCTGGCAGCCAGATCCAGTCCGA
GGAGTTTGAGCTGAATGACGAGAAGGCAGCAACAAGGATGGCACCTAACGGATGGAAGAGCAGACTGTTCAGGCACTCC
ACCCAGAAGAATCTGAAGAACTCTCAGATGTGCCAGAAGAGCCTGGACGTGGAAACCGATGGACTGGAGGCAAATGTGC
CACCCGTGAGCTTCCTGAAGGTGCTGAAGCTGAACAAGACAGAGTGGCCATATTTTGTGGTGGGCACCGTGTGCGCAATC
GCAAATGGCGGCCTGCAGCCAGCCTTCAGCGTGATCTTTTCCGAGATCATCGCCATCTTCGGCCCTGGCGACGATGCCGTG
AAGCAGCAGAAGTGTAACATCTTTTCCCTGATCTTCCTGTTTCTGGGCATCATCTCTTTCTTTACCTTCTTTCTGCAGGGCTTC
ACATTTGGCAAGGCCGGCGAGATCCTGACACGGAGACTGAGAAGCATGGCCTTCAAGGCCATGCTGAGGCAGGATATGT
CCTGGTTTGACGATCACAAGAACAGCACCGGCGCCCTGTCCACCAGACTGGCAACAGACGCAGCACAGGTGCAGGGAGC
AACCGGCACAAGGCTGGCCCTGATCGCCCAGAATATCGCCAACCTGGGCACAGGCATCATCATCTCTTTCATCTACGGCTG
GCAGCTGACCCTGCTGCTGCTGGCAGTGGTGCCAATCATCGCCGTGAGCGGCATCGTGGAGATGAAGCTGCTGGCCGGC
AATGCCAAGAGAGACAAGAAGGAGCTGGAGGCAGCAGGCAAGATCGCAACCGAGGCCATCGAGAACATCCGCACCGTG
GTGAGCCTGACACAGGAGCGGAAGTTCGAGTCCATGTATGTGGAGAAGCTGTATGGCCCTTACCGCAATTCCGTGCAGAA
GGCCCACATCTACGGCATCACCTTTTCCATCTCTCAGGCTTTCATGTATTTTTCTTACGCCGGCTGCTTCCGGTTTGGCGCCT
ATCTGATCGTGAACGGCCACATGAGGTTCCGCGATGTGATCCTGGTGTTCTCTGCCATCGTGTTTGGAGCAGTGGCCCTGG
GACACGCCTCCTCTTTTGCCCCTGACTATGCAAAGGCAAAGCTGTCCGCCGCACACCTGTTCATGCTGTTTGAGAGACAGC
CTCTGATCGATAGCTACTCCGAGGAGGGCCTGAAGCCAGACAAGTTCGAGGGCAATATCACATTCAACGAGGTGGTGTTT
AATTATCCAACCAGGGCCAACGTGCCCGTGCTGCAGGGCCTGAGCCTGGAGGTGAAGAAGGGACAGACACTGGCCCTGG
TGGGCAGCTCCGGATGCGGCAAGTCCACCGTGGTGCAGCTGCTGGAGAGATTCTACGACCCTCTGGCAGGCACCGTGCTG
CTGGATGGACAGGAGGCCAAGAAGCTGAATGTGCAGTGGCTGAGAGCCCAGCTGGGCATCGTGTCTCAGGAGCCAATCC
TGTTCGATTGTAGCATCGCCGAGAATATCGCCTACGGCGACAACTCTAGGGTGGTGAGCCAGGATGAGATCGTGAGCGCC
GCAAAGGCAGCAAACATCCACCCTTTTATCGAGACCCTGCCACACAAGTATGAGACACGCGTGGGCGACAAGGGCACCCA
GCTGTCCGGAGGACAGAAGCAGAGGATCGCAATCGCCCGCGCCCTGATCAGGCAGCCCCAGATCCTGCTGCTGGATGAA
GCCACCTCCGCCCTGGACACAGAGTCTGAGAAGGTGGTGCAGGAGGCCCTGGACAAAGCCCGCGAGGGACGGACATGTA
TTGTCATTGCTCACAGACTGAGCACCATCCAGAATGCCGACCTGATCGTGGTGTTCCAGAACGGCAGGGTGAAGGAGCAC
GGCACACACCAGCAGCTGCTGGCCCAGAAGGGCATCTACTTTTCTATGGTGAGCGTGCAGGCCGGCACCCAGAACCTGTA
G
**Codon-optimized sequence encoding MDR3 isoform A (co-MDR3(A)-2) (SEQ ID NO: 2)** 
ATGGATCTGGAAGCTGCCAAAAATGGCACAGCTTGGAGGCCCACCTCTGCTGAAGGGGATTTTGAGCTGGGCATCAGCA
GCAAGCAGAAGAGAAAAAAGACCAAGACAGTGAAGATGATTGGGGTGCTGACCCTGTTCAGATACTCTGACTGGCAGGA
CAAGCTGTTCATGAGCCTGGGCACCATCATGGCCATTGCTCATGGTTCTGGCCTGCCTCTGATGATGATTGTGTTTGGGGA
 
GATGACAGACAAGTTTGTGGACACAGCTGGCAACTTCAGCTTCCCTGTGAACTTCAGCCTGAGCCTGCTGAACCCTGGCAA
GATCCTGGAAGAGGAAATGACCAGATACGCCTACTACTACTCTGGCCTTGGAGCTGGTGTCCTGGTGGCTGCCTATATCCA
GGTGTCCTTTTGGACCCTGGCTGCTGGCAGACAGATCAGAAAGATCAGGCAGAAATTCTTCCATGCCATCCTGAGGCAAG
AGATTGGATGGTTTGACATCAATGACACCACAGAGCTGAACACCAGGCTGACAGATGACATCAGCAAGATCTCTGAAGGC
ATTGGGGACAAAGTGGGGATGTTCTTCCAGGCTGTGGCCACCTTCTTTGCTGGCTTCATTGTGGGCTTTATCAGAGGCTGG
AAACTGACCCTGGTCATCATGGCTATCAGCCCCATCCTGGGACTGTCTGCTGCTGTGTGGGCCAAAATCCTGTCTGCCTTCT
CTGACAAAGAACTGGCAGCCTATGCCAAGGCTGGTGCTGTGGCTGAAGAAGCCCTGGGAGCCATCAGAACAGTGATTGC
CTTTGGAGGGCAGAACAAAGAGCTGGAAAGATACCAGAAACACCTGGAAAATGCCAAAGAGATAGGCATCAAGAAGGC
CATCTCTGCCAACATCAGCATGGGCATTGCCTTTCTGCTCATCTATGCCAGCTATGCCCTGGCCTTTTGGTATGGCAGCACC
CTGGTTATCAGCAAAGAGTACACCATTGGCAACGCCATGACAGTGTTCTTCAGCATCCTGATTGGAGCCTTTTCTGTTGGCC
AGGCTGCCCCTTGCATTGATGCCTTTGCTAATGCCAGAGGGGCTGCTTATGTGATCTTTGACATTATTGACAACAACCCCAA
GATTGACAGCTTCTCTGAGAGAGGCCACAAGCCTGACAGCATCAAGGGCAACCTTGAGTTCAATGATGTGCACTTCAGCT
ACCCCAGCAGGGCCAATGTGAAAATCCTGAAGGGCCTGAACCTGAAGGTGCAGTCTGGACAGACAGTGGCCCTTGTGGG
ATCTTCTGGCTGTGGCAAGAGCACCACAGTGCAGCTGATCCAGAGACTGTATGACCCTGATGAGGGCACAATCAACATTG
ATGGCCAGGACATCAGAAACTTCAATGTGAACTACCTGAGGGAAATCATAGGGGTTGTGTCCCAAGAGCCTGTGCTGTTC
AGCACCACCATTGCTGAGAACATCTGCTATGGCAGGGGCAATGTCACAATGGATGAGATCAAGAAAGCTGTGAAAGAGG
CCAATGCCTATGAGTTCATCATGAAGCTGCCCCAGAAGTTTGACACCCTTGTTGGAGAAAGAGGGGCCCAGCTGTCTGGT
GGCCAGAAGCAGAGAATTGCCATTGCCAGGGCTCTTGTCAGAAACCCTAAGATCCTGCTGCTGGATGAGGCCACATCTGC
CCTGGATACAGAGTCTGAGGCAGAGGTGCAGGCTGCACTGGATAAGGCTAGAGAGGGAAGAACAACCATTGTGATTGCC
CACAGACTGAGCACAGTCAGAAATGCAGATGTGATTGCAGGCTTTGAGGATGGGGTCATAGTGGAACAGGGCAGCCACT
CTGAGCTGATGAAGAAAGAAGGGGTGTACTTCAAGCTGGTCAACATGCAGACCAGTGGCAGCCAGATCCAGTCTGAGGA
ATTTGAGCTGAATGATGAGAAGGCTGCCACCAGAATGGCCCCTAATGGCTGGAAGTCTAGGCTGTTTAGACACAGCACCC
AGAAGAACCTCAAGAACAGCCAGATGTGCCAGAAAAGCCTGGATGTTGAGACAGATGGCCTGGAAGCCAATGTGCCTCC
TGTGTCCTTCCTGAAAGTGCTGAAGCTGAACAAGACAGAGTGGCCCTACTTTGTTGTGGGCACAGTGTGTGCCATTGCAAA
TGGTGGCCTGCAGCCAGCTTTCTCTGTGATCTTCTCTGAAATCATTGCCATCTTTGGCCCTGGGGATGATGCTGTGAAGCA
GCAGAAGTGCAATATCTTTTCCCTGATCTTCCTGTTCCTGGGGATCATCAGTTTCTTCACATTCTTTCTGCAAGGCTTCACCT
TTGGCAAGGCTGGGGAGATCCTGACTAGAAGGCTGAGATCTATGGCCTTCAAAGCCATGCTGAGACAGGACATGTCTTGG
TTTGATGACCACAAGAACTCCACAGGGGCCCTGAGCACAAGACTGGCTACAGATGCTGCTCAGGTGCAGGGTGCTACAGG
CACTAGACTGGCCCTGATTGCACAGAACATTGCCAACCTTGGCACAGGCATCATCATTAGCTTCATCTATGGCTGGCAGCT
GACACTGCTCCTGCTGGCTGTTGTGCCCATCATTGCAGTGTCTGGCATTGTGGAAATGAAGTTGCTGGCTGGCAATGCCAA
GAGGGACAAGAAAGAGCTTGAGGCAGCAGGCAAGATTGCCACAGAGGCCATTGAGAATATCAGGACAGTGGTGTCTCTG
ACCCAAGAAAGAAAGTTTGAGTCTATGTATGTGGAAAAGCTGTATGGGCCCTATAGGAACTCTGTGCAAAAGGCCCACAT
CTATGGGATCACCTTCTCCATCAGCCAGGCTTTCATGTACTTTAGCTATGCTGGCTGCTTCAGATTTGGGGCCTACCTGATT
GTGAATGGCCACATGAGATTCAGGGATGTGATCCTGGTGTTTTCTGCCATTGTCTTTGGAGCTGTGGCTCTGGGCCATGCC
TCTAGCTTTGCCCCTGACTATGCCAAAGCCAAACTGTCTGCAGCCCACCTGTTCATGCTGTTTGAGAGACAGCCCCTGATTG
ACTCCTACTCTGAGGAAGGACTGAAGCCTGATAAGTTTGAGGGCAACATCACCTTCAATGAGGTGGTGTTCAACTACCCTA
CCAGAGCTAATGTGCCAGTGCTGCAGGGACTCTCCCTGGAAGTGAAGAAAGGGCAGACTCTGGCTCTTGTGGGCAGCTCA
GGATGTGGCAAGTCTACAGTGGTGCAGCTGCTGGAAAGGTTCTATGATCCCCTGGCAGGCACAGTGCTGTTGGATGGCCA
AGAGGCTAAAAAGCTGAATGTGCAGTGGCTGAGAGCACAGCTGGGAATTGTGTCTCAAGAACCCATCCTGTTTGACTGCA
GCATAGCAGAGAACATAGCCTATGGGGACAACTCCAGAGTGGTTTCTCAGGATGAGATTGTCTCTGCTGCCAAGGCAGCC
AACATTCACCCCTTCATTGAGACACTGCCCCACAAATATGAGACAAGAGTGGGAGACAAGGGAACCCAGCTCAGTGGTGG
ACAGAAACAAAGGATTGCTATAGCTAGGGCCCTGATCAGACAGCCTCAGATTCTGCTCCTTGATGAAGCAACCTCTGCTCT
GGACACTGAATCTGAGAAGGTGGTGCAAGAGGCCCTGGACAAAGCCAGAGAGGGTAGAACCTGCATTGTCATTGCTCAC
AGGCTGTCTACCATCCAGAATGCTGATCTGATTGTGGTGTTCCAGAATGGGAGAGTGAAAGAGCATGGCACCCACCAGCA
ACTGCTGGCCCAGAAGGGCATCTACTTCAGCATGGTGTCTGTGCAGGCAGGGACCCAGAACCTGTAA
**Synthetic poly A sequence (SEQ ID NO : 3)**
   AATAAAGACCTCTTATTTTCATTCATCAGGTGTGGTTGGTTTTTTTGTGTGGGGGC
**rAAV-MDR3-Aco 1(SEQ ID NO: 4)** 
CCTGCAGGCAGCTGCGCGCTCGCTCGCTCACTGAGGCCGCCCGGGCAAAGCCCGGGCGTCGGGCGACCTTTGGTCGCCCG
GCCTCAGTGAGCGAGCGAGCGCGCAGAGAGGGAGTGGCCAACTCCATCACTAGGGGTTCCTGCGGCCGCGAATTCCATG
GTACCAGGCATCAAGAcacgtgCGCCACCCCCTCCACCTTGGACACAGGACGCTGTGGTTTCTGAGCCAGGTACAATGACTC
CTTTCGGTAAGTGCAGTGGAAGCTGTACACTGCCCAGGCAAAGCGTCCGGGCAGCGTAGGCGGGCGACTCAGATCCCAG
CCAGTGGACTTAGCCCCTGTTTGCTCCTCCGATAACTGGGGTGACCTTGGTTAATATTCACCAGCAGCCTCCCCCGTTGCCC
CTCTGGATCCACTGCTTAAATACGGACGAGGACAGGGCCCTGTCTCCTCAGCTTCAGGCACCACCACTGACCTGGGACAGT
 
GAAGGCCTGTCGACGGATCCGCCGCCACCATGGATCTGGAGGCCGCCAAGAAcGGCACCGCcTGGAGACCCACAAGCGC
CGAGGGCGACTTCGAGCTGGGCATCAGCTCCAAGCAGAAGAGAAAGAAGACCAAGACAGTGAAGATGATCGGCGTGCT
GACACTGTTCAGGTACTCCGACTGGCAGGATAAGCTGTTTATGTCTCTGGGCACCATCATGGCAATCGCCCACGGCAGCG
GCCTGCCTCTGATGATGATCGTGTTCGGCGAGATGACCGACAAGTTTGTGGATACAGCCGGCAATTTCTCCTTTCCCGTGA
ACTTCTCTCTGAGCCTGCTGAACCCTGGCAAGATCCTGGAGGAGGAGATGACAAGATATGCCTACTATTACTCTGGCCTGG
GAGCAGGCGTGCTGGTGGCAGCATACATCCAGGTGAGCTTCTGGACCCTGGCAGCAGGCCGGCAGATCAGAAAGATCAG
GCAGAAGTTCTTTCACGCCATCCTGCGCCAGGAGATCGGCTGGTTTGACATCAATGATACCACAGAGCTGAACACCCGGCT
GACAGACGACATCTCTAAGATCAGCGAGGGCATCGGCGATAAAGTGGGCATGTTCTTTCAGGCCGTGGCCACATTCTTTG
CCGGCTTCATCGTGGGCTTTATCAGGGGCTGGAAGCTGACCCTGGTCATCATGGCCATCTCTCCAATCCTGGGCCTGAGCG
CCGCCGTGTGGGCAAAGATCCTGTCCGCCTTCTCTGACAAGGAGCTGGCCGCCTACGCCAAGGCAGGAGCAGTGGCAGA
GGAGGCCCTGGGCGCCATCCGCACCGTGATCGCCTTTGGCGGCCAGAATAAGGAGCTGGAGCGGTATCAGAAGCACCTG
GAGAACGCCAAGGAGATCGGCATCAAGAAGGCCATCTCCGCCAATATCTCTATGGGCATCGCCTTCCTGCTGATCTATGCC
AGCTACGCCCTGGCCTTTTGGTATGGCAGCACCCTGGTCATCAGCAAGGAGTACACCATCGGCAATGCCATGACAGTGTTC
TTTAGCATCCTGATCGGAGCCTTCTCCGTGGGACAGGCAGCACCATGCATCGACGCCTTCGCCAACGCCAGAGGCGCAGC
CTACGTGATCTTTGACATCATCGATAACAATCCAAAGATCGACTCCTTTTCTGAGCGGGGCCACAAGCCCGATTCCATCAAG
GGCAATCTGGAGTTCAACGACGTGCACTTTAGCTATCCCTCCCGCGCCAATGTGAAGATCCTGAAGGGCCTGAACCTGAA
GGTGCAGTCCGGACAGACAGTGGCCCTGGTGGGCTCTAGCGGATGCGGCAAGTCTACCACAGTGCAGCTGATCCAGCGG
CTGTATGACCCAGATGAGGGCACCATCAACATCGACGGCCAGGACATCCGCAACTTCAATGTGAACTACCTGCGGGAGAT
CATCGGCGTGGTGTCTCAGGAGCCCGTGCTGTTTAGCACCACAATCGCCGAGAATATCTGTTATGGCCGCGGCAACGTGA
CAATGGATGAGATCAAGAAGGCCGTGAAGGAGGCCAATGCCTACGAGTTCATCATGAAGCTGCCCCAGAAGTTTGACACC
CTGGTGGGAGAGAGAGGCGCCCAGCTGAGCGGAGGCCAGAAGCAGAGGATCGCCATCGCCAGAGCCCTGGTGAGGAAC
CCTAAGATCCTGCTGCTGGACGAGGCCACCTCCGCCCTGGATACAGAGTCTGAGGCAGAGGTGCAGGCCGCCCTGGATAA
GGCCCGCGAGGGACGGACCACAATCGTGATCGCCCACAGACTGAGCACAGTGAGGAATGCCGACGTGATCGCCGGCTTC
GAGGATGGCGTGATCGTGGAGCAGGGCAGCCACTCCGAGCTGATGAAGAAGGAGGGCGTGTACTTCAAGCTGGTGAAC
ATGCAGACCTCTGGCAGCCAGATCCAGTCCGAGGAGTTTGAGCTGAATGACGAGAAGGCAGCAACAAGGATGGCACCTA
ACGGATGGAAGAGCAGACTGTTCAGGCACTCCACCCAGAAGAATCTGAAGAACTCTCAGATGTGCCAGAAGAGCCTGGA
CGTGGAAACCGATGGACTGGAGGCAAATGTGCCACCCGTGAGCTTCCTGAAGGTGCTGAAGCTGAACAAGACAGAGTGG
CCATATTTTGTGGTGGGCACCGTGTGCGCAATCGCAAATGGCGGCCTGCAGCCAGCCTTCAGCGTGATCTTTTCCGAGATC
ATCGCCATCTTCGGCCCTGGCGACGATGCCGTGAAGCAGCAGAAGTGTAACATCTTTTCCCTGATCTTCCTGTTTCTGGGCA
TCATCTCTTTCTTTACCTTCTTTCTGCAGGGCTTCACATTTGGCAAGGCCGGCGAGATCCTGACACGGAGACTGAGAAGCAT
GGCCTTCAAGGCCATGCTGAGGCAGGATATGTCCTGGTTTGACGATCACAAGAACAGCACCGGCGCCCTGTCCACCAGAC
TGGCAACAGACGCAGCACAGGTGCAGGGAGCAACCGGCACAAGGCTGGCCCTGATCGCCCAGAATATCGCCAACCTGGG
CACAGGCATCATCATCTCTTTCATCTACGGCTGGCAGCTGACCCTGCTGCTGCTGGCAGTGGTGCCAATCATCGCCGTGAG
CGGCATCGTGGAGATGAAGCTGCTGGCCGGCAATGCCAAGAGAGACAAGAAGGAGCTGGAGGCAGCAGGCAAGATCGC
AACCGAGGCCATCGAGAACATCCGCACCGTGGTGAGCCTGACACAGGAGCGGAAGTTCGAGTCCATGTATGTGGAGAAG
CTGTATGGCCCTTACCGCAATTCCGTGCAGAAGGCCCACATCTACGGCATCACCTTTTCCATCTCTCAGGCTTTCATGTATTT
TTCTTACGCCGGCTGCTTCCGGTTTGGCGCCTATCTGATCGTGAACGGCCACATGAGGTTCCGCGATGTGATCCTGGTGTT
CTCTGCCATCGTGTTTGGAGCAGTGGCCCTGGGACACGCCTCCTCTTTTGCCCCTGACTATGCAAAGGCAAAGCTGTCCGC
CGCACACCTGTTCATGCTGTTTGAGAGACAGCCTCTGATCGATAGCTACTCCGAGGAGGGCCTGAAGCCAGACAAGTTCG
AGGGCAATATCACATTCAACGAGGTGGTGTTTAATTATCCAACCAGGGCCAACGTGCCCGTGCTGCAGGGCCTGAGCCTG
GAGGTGAAGAAGGGACAGACACTGGCCCTGGTGGGCAGCTCCGGATGCGGCAAGTCCACCGTGGTGCAGCTGCTGGAG
AGATTCTACGACCCTCTGGCAGGCACCGTGCTGCTGGATGGACAGGAGGCCAAGAAGCTGAATGTGCAGTGGCTGAGAG
CCCAGCTGGGCATCGTGTCTCAGGAGCCAATCCTGTTCGATTGTAGCATCGCCGAGAATATCGCCTACGGCGACAACTCTA
GGGTGGTGAGCCAGGATGAGATCGTGAGCGCCGCAAAGGCAGCAAACATCCACCCTTTTATCGAGACCCTGCCACACAA
GTATGAGACACGCGTGGGCGACAAGGGCACCCAGCTGTCCGGAGGACAGAAGCAGAGGATCGCAATCGCCCGCGCCCT
GATCAGGCAGCCCCAGATCCTGCTGCTGGATGAAGCCACCTCCGCCCTGGACACAGAGTCTGAGAAGGTGGTGCAGGAG
GCCCTGGACAAAGCCCGCGAGGGACGGACATGTATTGTCATTGCTCACAGACTGAGCACCATCCAGAATGCCGACCTGAT
CGTGGTGTTCCAGAACGGCAGGGTGAAGGAGCACGGCACACACCAGCAGCTGCTGGCCCAGAAGGGCATCTACTTTTCTA
TGGTGAGCGTGCAGGCCGGCACCCAGAACCTGTAGCATATGATATCAATAAAGACCTCTTATTTTCATTCATCAGGTGTGG
TTGGTTTTTTTGTGTGGGGGCTCGAGATCTGAGGAACCCCTAGTGATGGAGGCGGCCGCAGGAACCCCTAGTGATGGAGT
TGGCCACTCCCTCTCTGCGCGCTCGCTCGCTCACTGAGGCCGGGCGACCAAAGGTCGCCCGACGCCCGGGCTTTGCCCGG
GCGGCCTCAGTGAGCGAGCGAGCGCGCAGCTGCCTGCAGG
**rAAV-MDR3-Aco 2 (SEQ ID NO: 5)** 
CCTGCAGGCAGCTGCGCGCTCGCTCGCTCACTGAGGCCGCCCGGGCAAAGCCCGGGCGTCGGGCGACCTTTGGTCGCCCG
GCCTCAGTGAGCGAGCGAGCGCGCAGAGAGGGAGTGGCCAACTCCATCACTAGGGGTTCCTGCGGCCGCGAATTCCATG
GTACCAGGCATCAAGACACGTGCGCCACCCCCTCCACCTTGGACACAGGACGCTGTGGTTTCTGAGCCAGGTACAATGACT
 
CCTTTCGGTAAGTGCAGTGGAAGCTGTACACTGCCCAGGCAAAGCGTCCGGGCAGCGTAGGCGGGCGACTCAGATCCCA
GCCAGTGGACTTAGCCCCTGTTTGCTCCTCCGATAACTGGGGTGACCTTGGTTAATATTCACCAGCAGCCTCCCCCGTTGCC
CCTCTGGATCCACTGCTTAAATACGGACGAGGACAGGGCCCTGTCTCCTCAGCTTCAGGCACCACCACTGACCTGGGACAG
TGAAGGCCTGTCGACGGATCCGCCGCCACCATGGATCTGGAAGCTGCCAAAAATGGCACAGCTTGGAGGCCCACCTCTGC
TGAAGGGGATTTTGAGCTGGGCATCAGCAGCAAGCAGAAGAGAAAAAAGACCAAGACAGTGAAGATGATTGGGGTGCT
GACCCTGTTCAGATACTCTGACTGGCAGGACAAGCTGTTCATGAGCCTGGGCACCATCATGGCCATTGCTCATGGTTCTGG
CCTGCCTCTGATGATGATTGTGTTTGGGGAGATGACAGACAAGTTTGTGGACACAGCTGGCAACTTCAGCTTCCCTGTGAA
CTTCAGCCTGAGCCTGCTGAACCCTGGCAAGATCCTGGAAGAGGAAATGACCAGATACGCCTACTACTACTCTGGCCTTGG
AGCTGGTGTCCTGGTGGCTGCCTATATCCAGGTGTCCTTTTGGACCCTGGCTGCTGGCAGACAGATCAGAAAGATCAGGC
AGAAATTCTTCCATGCCATCCTGAGGCAAGAGATTGGATGGTTTGACATCAATGACACCACAGAGCTGAACACCAGGCTG
ACAGATGACATCAGCAAGATCTCTGAAGGCATTGGGGACAAAGTGGGGATGTTCTTCCAGGCTGTGGCCACCTTCTTTGCT
GGCTTCATTGTGGGCTTTATCAGAGGCTGGAAACTGACCCTGGTCATCATGGCTATCAGCCCCATCCTGGGACTGTCTGCT
GCTGTGTGGGCCAAAATCCTGTCTGCCTTCTCTGACAAAGAACTGGCAGCCTATGCCAAGGCTGGTGCTGTGGCTGAAGA
AGCCCTGGGAGCCATCAGAACAGTGATTGCCTTTGGAGGGCAGAACAAAGAGCTGGAAAGATACCAGAAACACCTGGAA
AATGCCAAAGAGATAGGCATCAAGAAGGCCATCTCTGCCAACATCAGCATGGGCATTGCCTTTCTGCTCATCTATGCCAGC
TATGCCCTGGCCTTTTGGTATGGCAGCACCCTGGTTATCAGCAAAGAGTACACCATTGGCAACGCCATGACAGTGTTCTTC
AGCATCCTGATTGGAGCCTTTTCTGTTGGCCAGGCTGCCCCTTGCATTGATGCCTTTGCTAATGCCAGAGGGGCTGCTTATG
TGATCTTTGACATTATTGACAACAACCCCAAGATTGACAGCTTCTCTGAGAGAGGCCACAAGCCTGACAGCATCAAGGGCA
ACCTTGAGTTCAATGATGTGCACTTCAGCTACCCCAGCAGGGCCAATGTGAAAATCCTGAAGGGCCTGAACCTGAAGGTG
CAGTCTGGACAGACAGTGGCCCTTGTGGGATCTTCTGGCTGTGGCAAGAGCACCACAGTGCAGCTGATCCAGAGACTGTA
TGACCCTGATGAGGGCACAATCAACATTGATGGCCAGGACATCAGAAACTTCAATGTGAACTACCTGAGGGAAATCATAG
GGGTTGTGTCCCAAGAGCCTGTGCTGTTCAGCACCACCATTGCTGAGAACATCTGCTATGGCAGGGGCAATGTCACAATG
GATGAGATCAAGAAAGCTGTGAAAGAGGCCAATGCCTATGAGTTCATCATGAAGCTGCCCCAGAAGTTTGACACCCTTGT
TGGAGAAAGAGGGGCCCAGCTGTCTGGTGGCCAGAAGCAGAGAATTGCCATTGCCAGGGCTCTTGTCAGAAACCCTAAG
ATCCTGCTGCTGGATGAGGCCACATCTGCCCTGGATACAGAGTCTGAGGCAGAGGTGCAGGCTGCACTGGATAAGGCTA
GAGAGGGAAGAACAACCATTGTGATTGCCCACAGACTGAGCACAGTCAGAAATGCAGATGTGATTGCAGGCTTTGAGGA
TGGGGTCATAGTGGAACAGGGCAGCCACTCTGAGCTGATGAAGAAAGAAGGGGTGTACTTCAAGCTGGTCAACATGCAG
ACCAGTGGCAGCCAGATCCAGTCTGAGGAATTTGAGCTGAATGATGAGAAGGCTGCCACCAGAATGGCCCCTAATGGCTG
GAAGTCTAGGCTGTTTAGACACAGCACCCAGAAGAACCTCAAGAACAGCCAGATGTGCCAGAAAAGCCTGGATGTTGAG
ACAGATGGCCTGGAAGCCAATGTGCCTCCTGTGTCCTTCCTGAAAGTGCTGAAGCTGAACAAGACAGAGTGGCCCTACTTT
GTTGTGGGCACAGTGTGTGCCATTGCAAATGGTGGCCTGCAGCCAGCTTTCTCTGTGATCTTCTCTGAAATCATTGCCATCT
TTGGCCCTGGGGATGATGCTGTGAAGCAGCAGAAGTGCAATATCTTTTCCCTGATCTTCCTGTTCCTGGGGATCATCAGTTT
CTTCACATTCTTTCTGCAAGGCTTCACCTTTGGCAAGGCTGGGGAGATCCTGACTAGAAGGCTGAGATCTATGGCCTTCAA
AGCCATGCTGAGACAGGACATGTCTTGGTTTGATGACCACAAGAACTCCACAGGGGCCCTGAGCACAAGACTGGCTACAG
ATGCTGCTCAGGTGCAGGGTGCTACAGGCACTAGACTGGCCCTGATTGCACAGAACATTGCCAACCTTGGCACAGGCATC
ATCATTAGCTTCATCTATGGCTGGCAGCTGACACTGCTCCTGCTGGCTGTTGTGCCCATCATTGCAGTGTCTGGCATTGTGG
AAATGAAGTTGCTGGCTGGCAATGCCAAGAGGGACAAGAAAGAGCTTGAGGCAGCAGGCAAGATTGCCACAGAGGCCA
TTGAGAATATCAGGACAGTGGTGTCTCTGACCCAAGAAAGAAAGTTTGAGTCTATGTATGTGGAAAAGCTGTATGGGCCC
TATAGGAACTCTGTGCAAAAGGCCCACATCTATGGGATCACCTTCTCCATCAGCCAGGCTTTCATGTACTTTAGCTATGCTG
GCTGCTTCAGATTTGGGGCCTACCTGATTGTGAATGGCCACATGAGATTCAGGGATGTGATCCTGGTGTTTTCTGCCATTG
TCTTTGGAGCTGTGGCTCTGGGCCATGCCTCTAGCTTTGCCCCTGACTATGCCAAAGCCAAACTGTCTGCAGCCCACCTGTT
CATGCTGTTTGAGAGACAGCCCCTGATTGACTCCTACTCTGAGGAAGGACTGAAGCCTGATAAGTTTGAGGGCAACATCA
CCTTCAATGAGGTGGTGTTCAACTACCCTACCAGAGCTAATGTGCCAGTGCTGCAGGGACTCTCCCTGGAAGTGAAGAAA
GGGCAGACTCTGGCTCTTGTGGGCAGCTCAGGATGTGGCAAGTCTACAGTGGTGCAGCTGCTGGAAAGGTTCTATGATCC
CCTGGCAGGCACAGTGCTGTTGGATGGCCAAGAGGCTAAAAAGCTGAATGTGCAGTGGCTGAGAGCACAGCTGGGAATT
GTGTCTCAAGAACCCATCCTGTTTGACTGCAGCATAGCAGAGAACATAGCCTATGGGGACAACTCCAGAGTGGTTTCTCAG
GATGAGATTGTCTCTGCTGCCAAGGCAGCCAACATTCACCCCTTCATTGAGACACTGCCCCACAAATATGAGACAAGAGTG
GGAGACAAGGGAACCCAGCTCAGTGGTGGACAGAAACAAAGGATTGCTATAGCTAGGGCCCTGATCAGACAGCCTCAGA
TTCTGCTCCTTGATGAAGCAACCTCTGCTCTGGACACTGAATCTGAGAAGGTGGTGCAAGAGGCCCTGGACAAAGCCAGA
GAGGGTAGAACCTGCATTGTCATTGCTCACAGGCTGTCTACCATCCAGAATGCTGATCTGATTGTGGTGTTCCAGAATGGG
AGAGTGAAAGAGCATGGCACCCACCAGCAACTGCTGGCCCAGAAGGGCATCTACTTCAGCATGGTGTCTGTGCAGGCAG
GGACCCAGAACCTGTAACATATGATATCAATAAAGACCTCTTATTTTCATTCATCAGGTGTGGTTGGTTTTTTTGTGTGGGG
GCTCGAGATCTGAGGAACCCCTAGTGATGGAGGCGGCCGCAGGAACCCCTAGTGATGGAGTTGGCCACTCCCTCTCTGCG
CGCTCGCTCGCTCACTGAGGCCGGGCGACCAAAGGTCGCCCGACGCCCGGGCTTTGCCCGGGCGGCCTCAGTGAGCGAG
CGAGCGCGCAGCTGCCTGCAGG
**Alpha 1 antitrypsin promoter (SEQ ID NO: 6)** 
CGCCACCCCCTCCACCTTGGACACAGGACGCTGTGGTTTCTGAGCCAGGTACAATGACTCCTTTCGGTAAGTGCAGTGGAA
GCTGTACACTGCCCAGGCAAAGCGTCCGGGCAGCGTAGGCGGGCGACTCAGATCCCAGCCAGTGGACTTAGCCCCTGTTT
GCTCCTCCGATAACTGGGGTGACCTTGGTTAATATTCACCAGCAGCCTCCCCCGTTGCCCCTCTGGATCCACTGCTTAAATA
CGGACGAGGACA
**Human minimal bile salt export pump (ABCB11)gene promoter (SEQ ID NO: 7)** 
TTCCCAAGCACACTCTGTGTTTGGGGTTATTGCTCTGAGTATGTTTCTCGTATGTCACTGAACTGTGCTTGGGCTGCCCTTA
GGGACATTGATCCTTAGGCAAATAGATAATGTTCTTGAAAAAGTTTGAATTCTGTTCAGTGCT
**Mouse minimal Bile Salt export pump (ABCB11) gene promoter (SEQ ID NO: 8)**

GGTTCCTGCTTTGAGTATGTTCGACCTTTCCTCTCATGTCACTGAACTGTGCTAGATCTGGACTTTAGGCCATTGACCTATAA
GCAAATAGATAGTGTTCTTAAAAAAGCCTGATTTCTGTTCAATGCTTTATTACCATGAAAAC

## Claims

1. A nucleic acid construct comprising a transgene encoding MDR3 isoform A, said transgene is the sequence SEQ ID NO: 1 or a sequence having at least 90 % of identity with SEQ ID NO: 1.

2. The nucleic acid construct of claim 1 further comprising a liver-specific promoter, preferably an alpha-1-antitrypsin promoter or a bile salt-inducible promoter.

3. The nucleic acid construct of claim 1 or 2 further comprising a polyadenylation signal sequence, notably a synthetic polyadenylation signal sequence having sequence SEQ ID NO: 3.

4. The nucleic acid construct according to any one of claims 1 to 3 further comprising a 5'ITR and a 3'ITR sequences, preferably a 5'ITR and a 3'ITR sequences of an adeno-associated virus, more preferably a 5'ITR and a 3'ITR sequences from the AAV2 serotype.

5. The nucleic acid construct according to any one of claims 1 to 4 comprising nucleic acid sequence SEQ ID NO: 4 or a nucleic acid sequence having at least 90% of identity with SEQ ID NO: 4.

6. An expression vector comprising a nucleic acid construct according to any of claims 1 to 5.

7. The expression vector of claim 6 wherein said vector is a viral vector, preferably an adeno-associated viral (AAV) vector.

8. A viral particle comprising a nucleic acid construct according to any of claims 1 to 5 or expression vector of claim 6 or 7.

9. An AAV particle comprising a nucleic acid construct according to any of claims 1 to 5 or expression vector of claim 6 or 7 and preferably comprising capsid proteins of adeno-associated virus such as capsid proteins selected from the group consisting of: AAV3 type 3A, AAV3 type 3B, NP40, NP59, NP84, LK03, AAV3-ST, Anc80 and AAV8 serotype.

10. An isolated host cell comprising a nucleic acid construct according to any of claims 1 to 5 or an expression vector of claims 6 or 7 or an isolated host cell transduced with a viral particle of claim 8 or 9.

11. A Pharmaceutical composition comprising a nucleic acid construct according to any of claims 1 to 5, a vector of claim 6 or 7, a viral particle of claim 8 or 9 or a host cell of claim 10 and a pharmaceutically acceptable excipient.

12. A nucleic acid construct according to any of claims 1 to 5, a vector of claim 6 or 7, a viral particle of claim 8 or 9, a host cell of claim 10 or a pharmaceutical composition of claim 11 for its use as a medicament in a subject in need thereof.

13. A nucleic acid construct according to any of claims 1 to 5, a vector of claim 6 or 7, a viral particle of claim 8 or 9, a host cell of claim 10 or a pharmaceutical composition of claim 11 for its use for the prevention and/or treatment of progressive familiar Intrahepatic Cholestasis Type 3 (PFIC3) in a subject in need thereof, preferably said subject is a neonate, an infant, a child or an adult, more preferably a neonate, an infant or a child, more preferably a neonate or an infant.

14. A method of producing viral particles according to claim 8 or 9, comprising the steps of:
a) culturing a host cell comprising a nucleic acid construct or an expression vector according to claim 10 in a culture medium, and
b) harvesting the viral particles from the cell culture supernatant and/or inside the cells.

15. A kit comprising a nucleic acid construct according to any of claims 1 to 5, a vector of claim 6 or 7, a viral particle of claims 8 or 9, a host cell of claim 10 or a pharmaceutical composition of claim 11, in one or more containers, optionally further comprising instructions or packaging materials.

## Patentansprüche

1. Nukleinsäurekonstrukt, umfassend ein Transgen, das für MDR3-Isoform A kodiert, wobei das Transgen die Sequenz SEQ ID NO: 1 oder eine Sequenz mit mindestens 90 % Identität zu SEQ ID NO: 1 ist.

2. Nukleinsäurekonstrukt nach Anspruch 1, ferner umfassend einen leberspezifischen Promotor, vorzugsweise einen alpha-1-Antitrypsin-Promotor oder einen Gallensalz-induzierbaren Promotor.

3. Nukleinsäurekonstrukt nach Anspruch 1 oder 2, ferner umfassend eine Polyadenylierungssignalsequenz, insbesondere eine synthetische Polyadenylierungssignalsequenz mit der Sequenz SEQ ID NO: 3.

4. Nukleinsäurekonstrukt nach einem der Ansprüche 1 bis 3, ferner umfassend eine 5'ITR- und eine 3'ITR-Sequenz, vorzugsweise eine 5'ITR- und eine 3'ITR-Sequenz eines Adeno-assoziierten Virus, besonders bevorzugt eine 5'ITR- und eine 3'ITR-Sequenz des AAV2-Serotyps.

5. Nukleinsäurekonstrukt nach einem der Ansprüche 1 bis 4, umfassend die Nukleinsäuresequenz SEQ ID NO: 4 oder eine Nukleinsäuresequenz mit mindestens 90% Identität zu SEQ ID NO: 4.

6. Expressionsvektor, umfassend ein Nukleinsäurekonstrukt nach einem der Ansprüche 1 bis 5.

7. Expressionsvektor nach Anspruch 6, wobei der Vektor ein viraler Vektor, vorzugsweise ein Adeno-assoziierter viraler (AAV) Vektor ist.

8. Viruspartikel, umfassend ein Nukleinsäurekonstrukt nach einem der Ansprüche 1 bis 5 oder einen Expressionsvektor nach Anspruch 6 oder 7.

9. AAV-Partikel, umfassend ein Nukleinsäurekonstrukt nach einem der Ansprüche 1 bis 5 oder einen Expressionsvektor nach Anspruch 6 oder 7 und vorzugsweise umfassend Capsidproteine eines Adeno-assoziierten Virus, wie etwa Capsidproteine, die ausgewählt sind aus der Gruppe bestehend aus: AAV3 Typ 3A, AAV3 Typ 3B, NP40, NP59, NP84, LK03, AAV3-ST, Anc80 und AAV 8 Serotyp.

10. Isolierte Wirtszelle, die ein Nukleinsäurekonstrukt nach einem der Ansprüche 1 bis 5 oder einen Expressionsvektor nach Anspruch 6 oder 7 umfasst, oder isolierte Wirtszelle, die mit einem Viruspartikel nach Anspruch 8 oder 9 transduziert wurde.

11. Pharmazeutische Zusammensetzung, umfassend ein Nukleinsäurekonstrukt nach einem der Ansprüche 1 bis 5, einen Vektor nach Anspruch 6 oder 7, ein Viruspartikel nach Anspruch 8 oder 9 oder eine Wirtszelle nach Anspruch 10 und einen pharmazeutisch annehmbaren Hilfsstoff.

12. Nukleinsäurekonstrukt nach einem der Ansprüche 1 bis 5, Vektor nach Anspruch 6 oder 7, Viruspartikel nach Anspruch 8 oder 9, Wirtszelle nach Anspruch 10 oder pharmazeutische Zusammensetzung nach Anspruch 11 zur Verwendung als Arzneimittel bei einem Individuum, das dessen bedarf.

13. Nukleinsäurekonstrukt nach einem der Ansprüche 1 bis 5, Vektor nach Anspruch 6 oder 7, Viruspartikel nach Anspruch 8 oder 9, Wirtszelle nach Anspruch 10 oder pharmazeutische Zusammensetzung nach Anspruch 11 zur Verwendung für die Vorbeugung und/oder Behandlung von progressiver familiärer intrahepatischer Cholestase Typ 3 (PFIC3) in einem Individuum, das dessen bedarf, wobei das Individuum vorzugsweise ein Neugeborenes, ein Säugling, ein Kind oder ein Erwachsener ist, stärker bevorzugt ein Neugeborenes, ein Säugling oder ein Kind, stärker bevorzugt ein Neugeborenes oder ein Säugling.

14. Verfahren zur Herstellung von Viruspartikeln nach Anspruch 8 oder 9, umfassend die Schritte:
a) Kultivieren einer Wirtszelle, die ein Nukleinsäurekonstrukt oder einen Expressionsvektor nach Anspruch 10 umfasst, in einem Kulturmedium und
b) Ernten der Viruspartikel aus dem Zellkulturüberstand und/oder innerhalb der Zellen.

15. Kit, umfassend ein Nukleinsäurekonstrukt nach einem der Ansprüche 1 bis 5, einen Vektor nach Anspruch 6 oder 7, ein Viruspartikel nach Anspruch 8 oder 9, eine Wirtszelle nach Anspruch 10 oder eine pharmazeutische Zusammensetzung nach Anspruch 11, in einem oder mehreren Behältern, gegebenenfalls ferner umfassend Anweisungen oder Verpackungsmaterialien.

## Revendications

1. Construction d'acide nucléique comprenant un transgène codant pour l'isoforme A de MDR3, ledit transgène est la séquence SEQ ID NO : 1 ou une séquence ayant au moins 90 % d'identité avec SEQ ID NO : 1.

2. Construction d'acide nucléique selon la revendication 1, comprenant en outre un promoteur spécifique du foie, de préférence un promoteur d'alpha-1-antitrypsine ou un promoteur inductible par les sels biliaires.

3. Construction d'acide nucléique selon la revendication 1 ou 2, comprenant en outre une séquence signal de polyadénylation, notamment une séquence signal de polyadénylation synthétique ayant la séquence SEQ ID NO : 3.

4. Construction d'acide nucléique selon l'une quelconque des revendications 1 à 3, comprenant en outre une séquence ITR en 5' et une séquence ITR en 3', de préférence une séquence ITR en 5' et une séquence ITR en 3' d'un virus adéno-associé, de manière davantage préférée une séquence ITR en 5' et une séquence ITR en 3' provenant du sérotype AAV2.

5. Construction d'acide nucléique selon l'une quelconque des revendications 1 à 4 comprenant la séquence d'acide nucléique SEQ ID NO : 4 ou une séquence d'acide nucléique ayant au moins 90 % d'identité avec SEQ ID NO : 4.

6. Vecteur d'expression comprenant une construction d'acide nucléique selon l'une quelconque des revendications 1 à 5.

7. Vecteur d'expression selon la revendication 6, dans lequel ledit vecteur est un vecteur viral, de préférence un vecteur viral adéno-associé (AAV).

8. Particule virale comprenant une construction d'acide nucléique selon l'une quelconque des revendications 1 à 5 ou un vecteur d'expression selon la revendication 6 ou 7.

9. Particule d'AAV comprenant une construction d'acide nucléique selon l'une quelconque des revendications 1 à 5 ou un vecteur d'expression selon la revendication 6 ou 7 et comprenant de préférence des protéines de capside de virus adéno-associé telles que les protéines de capside sélectionnées dans le groupe constitué de : AAV3 type 3A, AAV3 type 3B, NP40, NP59, NP84, LK03, AAV3-ST, Anc80 et sérotype AAV8.

10. Cellule hôte isolée comprenant une construction d'acide nucléique selon l'une quelconque des revendications 1 à 5 ou un vecteur d'expression selon les revendications 6 ou 7 ou une cellule hôte isolée transduite avec une particule virale selon la revendication 8 ou 9.

11. Composition pharmaceutique comprenant une construction d'acide nucléique selon l'une quelconque des revendications 1 à 5, un vecteur selon la revendication 6 ou 7, une particule virale selon la revendication 8 ou 9 ou une cellule hôte selon la revendication 10 et un excipient pharmaceutiquement acceptable.

12. Construction d'acide nucléique selon l'une quelconque des revendications 1 à 5, vecteur selon la revendication 6 ou 7, particule virale selon la revendication 8 ou 9, cellule hôte selon la revendication 10 ou composition pharmaceutique selon la revendication 11 pour son utilisation comme médicament chez un sujet en ayant besoin.

13. Construction d'acide nucléique selon l'une quelconque des revendications 1 à 5, vecteur selon la revendication 6 ou 7, particule virale selon la revendication 8 ou 9, cellule hôte selon la revendication 10 ou composition pharmaceutique selon la revendication 11 pour son utilisation pour la prévention et/ou le traitement de la cholestase intrahépatique familiale progressive de type 3 (PFIC3) chez un sujet en ayant besoin, de préférence ledit sujet est un nouveau-né, un nourrisson, un enfant ou un adulte, de manière davantage préférée un nouveau-né, un nourrisson ou un enfant, de manière davantage préférée un nouveau-né ou un nourrisson.

14. Procédé de production de particules virales selon la revendication 8 ou 9, comprenant les étapes suivantes :
a) la mise en culture d'une cellule hôte comprenant une construction d'acide nucléique ou un vecteur d'expression selon la revendication 10 dans un milieu de culture, et
b) la collecte des particules virales à partir du surnageant de culture cellulaire et/ou à l'intérieur des cellules.

15. Kit comprenant une construction d'acide nucléique selon l'une quelconque des revendications 1 à 5, un vecteur selon la revendication 6 ou 7, une particule virale selon la revendication 8 ou 9, une cellule hôte selon la revendication 10 ou une composition pharmaceutique selon la revendication 11, dans une ou plusieurs récipients, facultativement comprenant en outre des instructions ou des matériaux d'emballage.
